(19) Europäisches Patentamt / European Patent Office / Office européen des brevets

(11) EP 4 400 094 A1

(12) **EUROPEAN PATENT APPLICATION**
published in accordance with Art. 153(4) EPC

(43) Date of publication:
**17.07.2024 Bulletin 2024/29**

(21) Application number: **22867718.3**

(22) Date of filing: **07.09.2022**

(51) International Patent Classification (IPC):
***A61K 9/00*** *(2006.01)* ***A61K 47/28*** *(2006.01)*
***A61K 38/00*** *(2006.01)*

(52) Cooperative Patent Classification (CPC):
**A61K 9/00; A61K 38/00; A61K 47/28**

(86) International application number:
**PCT/KR2022/013497**

(87) International publication number:
**WO 2023/038450 (16.03.2023 Gazette 2023/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.09.2021 KR 20210118832**

(71) Applicant: **D&D Pharmatech Inc.**
**Seongnam-si, Gyeonggi-do 13486 (KR)**

(72) Inventors:
- **PARK, Eun Ji**
  **Seoul 06503 (KR)**
- **CHOI, Ji Young**
  **Suwon-si, Gyeonggi-do 16225 (KR)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

(54) **PHARMACEUTICAL COMPOSITION COMPRISING LARGE PHYSIOLOGICALLY ACTIVE SUBSTANCE AND EXCIPIENT**

(57) The present invention relates to a pharmaceutical composition that efficiently increases the absorption rate of a large physiologically active substance in the body; and specifically to a pharmaceutical composition comprising: (i) a large physiologically active substance; and (ii) excipient A comprising a bile acid derivative or (iii) excipient B comprising a compound having CYP450 inhibition, an antioxidant effect, or gastrointestinal enzyme activity inhibitory effect, and a method for preparing same.



Processed by Luminess, 75001 PARIS (FR)

(Cont. next page)

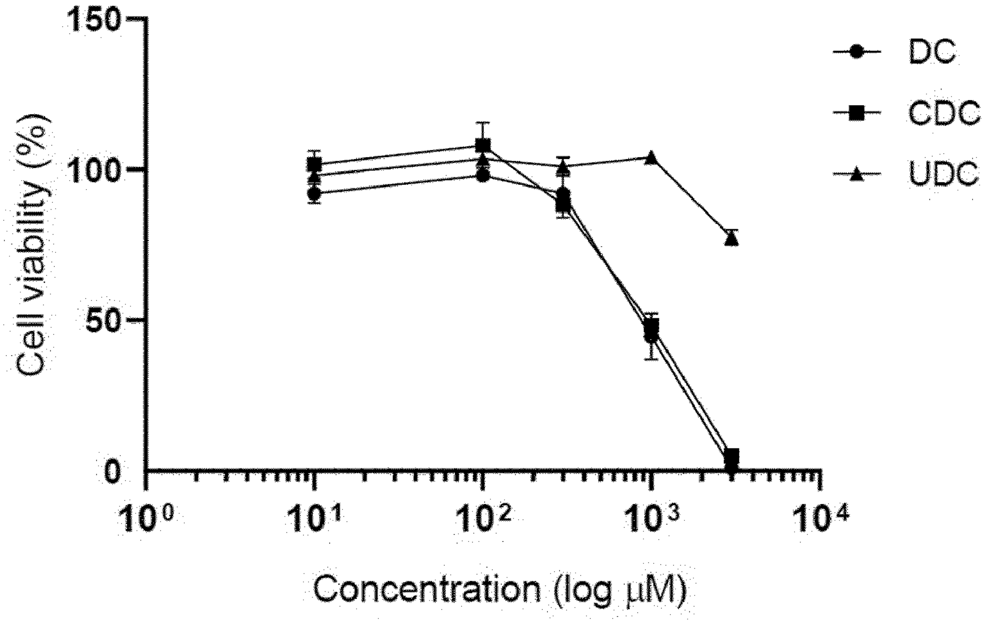
150
100
50
0
Cell viability (%)
10⁰
10¹
10²
10³
10⁴
Concentration (log μM)
DC
CDC
UDC

[FIG. 1]

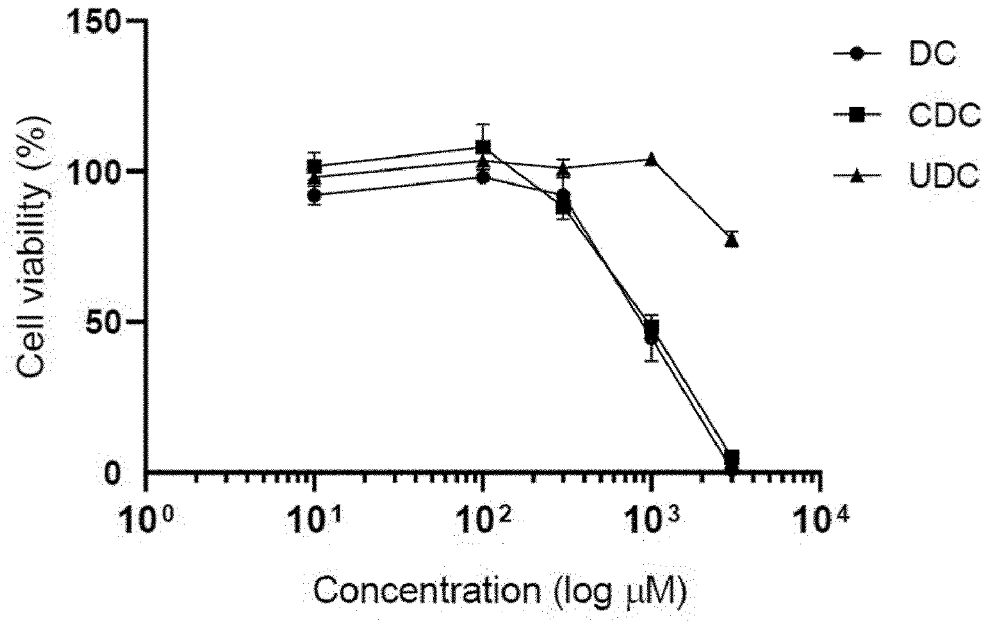
150
100
50
0
Cell viability (%)
10⁰
10¹
10²
10³
10⁴
Concentration (log μM)
DC
CDC
UDC

## Description

[Technical Field]

The present invention relates to a formulation for increasing the oral bioavailability of macromolecules. In particular, the present invention relates to a pharmaceutical composition comprising (i) a large physiologically active substance; and (ii) excipient A comprising a bile acid derivative; or (iii) excipient B comprising a compound or a derivative thereof having CYP450 inhibition, antioxidant effect, or gastrointestinal enzyme activity inhibition effect; and to a method of producing the same.

[Background Art]

In modern society relative to current eating habits, a large number of people are suffering from lifestyle diseases such as cardiovascular disease, diabetes, and osteoporosis due to changes in eating habits. Diabetes, a type of lifestyle disease, is spreading worldwide, and it is estimated that more than 1.5 million people die from diabetes every year. The global diabetes market is expected to grow from $80.15 billion in 2016 at a CAGR of 12.4%, reaching $161.69 billion in 2022. In addition, the market size is growing in all fields, including osteoporosis and cardiovascular diseases, due to aging resulting from medical advancements.

In terms of drug delivery methods, they are largely divided into oral and parenteral dosage forms; a large number of oral dosage forms of drugs are being developed considering cost, patient administration convenience, ease of production, and the like. From the perspective of treatment compliance of patients, the oral administration of therapeutic agents is generally considered to be a superior administration route than parenteral administration, and this is particularly true when the nature of the therapeutic agent or the nature of the condition to be treated requires multiple daily administrations of the therapeutic agent. However, despite this, macromolecules, such as polypeptides and polysaccharides, are known to be very difficult to successfully administer orally; accordingly, macromolecules have been administered parenterally, for example, by subcutaneous, intramuscular or intravenous injection. Therefore, it is highly desirable to provide a formulation that enhances the oral bioavailability of macromolecules to a range that allows oral administration of such macromolecules. In the case of diabetes treatments for which the market size is large and the growth potential is high, oral dosage forms have been developed by many companies. Specifically, the DPP-4 inhibitor series includes MSD's Januvia, Boehringer Ingelheim's Trajenta, LG Chem's Zemiglo, Novartis' Galvus, and AstraZeneca's Onglyza and Kombiglyze; the SGLT-2 inhibitor series includes AstraZeneca's Farxiga, Xigduo, and Boehringer Ingelheim's Jardiance, and the TZD series includes Takeda's Actos and Chong Kun Dang's Duvier. Additionally, there is Novo Nordisk's Rybelsus, which is an oral GLP-1. However, among the drugs above, DPP-4 inhibitors are sitagliptin, vildagliptin, saxagliptin, linagliptin, alogliptin and gemigliptin; SGLT-2 inhibitors are Dapagliflozin, Ipragliflozin and Empagliflozin; and TZD is thiazolidinedione; and these are not macromolecular drugs.

The mass production of macromolecular drugs, such as insulin, heparin, calcitonin, interferon, and growth hormone, has become possible due to advances in electronic manipulation and biological processing, but macromolecular drugs have problems relative to their large molecular weight, making it difficult for them to pass through the gastrointestinal membrane or being easily broken down by digestive enzymes, and thus oral administration is limited, and treatment is consequently carried out by means of methods such as intravenous injection and intramuscular injection. These forms of administration, such as intravenous and intramuscular injections enter the systemic circulation directly and thus have the advantage of high bioavailability and rapid onset of drug effects, but the onset of drug effects due to immediate administration of drugs into the blood increases the risk of side effects. Furthermore, administration by injection is associated with low patient compliance due to pain and discomfort. There are also cases wherein self-administration is impossible or the administration must be performed in a hospital. In the latter case, when the half-life of the drug is short and repeated administration is required, such can also be a troublesome problem, and when hospital treatment or hospitalization is required, the increased burden on society can be problematic. To solve this problem, research is being conducted domestically and internationally to manufacture oral drugs, but there is a problem that macromolecular drugs generally have a bioavailability of only 0 to 2% when administered orally, making the possibility of success low.

Recently, many studies have been conducted on the oral absorption of macromolecular drugs, but the situation is such that the demand for oral drugs is increasing and that the supply thereof is insufficient. The present inventor aims to solve this problem by using a bile acid-based excipient.

[Detailed Description of the Invention]

[Problem to be Solved]

In order to solve the above problem, the present invention aims to efficiently increase the body absorption rate

of the large physiologically active substance by mixing the large physiologically active substance with a specific excipient to prepare a pharmaceutical composition having an excellent oral absorption rate.

[Means of Solving the Problem]

In order to achieve the above objective, the present invention provides a pharmaceutical composition comprising (i) a large physiologically active substance; and (ii) excipient A comprising a bile acid derivative; or (iii) excipient B comprising a compound or a derivative thereof having CYP450 inhibition, antioxidant effect, or gastrointestinal enzyme activity inhibition effect.

In one aspect of the present invention, the (i) large physiologically active substance is a polypeptide, protein, polysaccharide, nucleotide, or an analog thereof.

In one aspect of the present invention, in (ii) excipient A, the bile acid derivative is one or more selected from the group consisting of glycocholic acid, glycocholicchenodeoxycholic acid, taurocholic acid, deoxycholic acid, taurodeoxycholic acid, cholic acid, chenodeoxycholic acid, ursodeoxycholic acid, lithocholic acid, dehydrocholic acid, and pharmaceutically acceptable salts thereof. Furthermore, in one aspect of the present invention, the bile acid derivative is two or more types selected from the above structural group.

Furthermore, in one aspect of the present invention, in (ii) excipient A, the bile acid derivative is one or more selected from the group consisting of glycocholic acid, taurocholic acid, deoxycholic acid, cholic acid, chenodeoxycholic acid, ursodeoxycholic acid and pharmaceutically acceptable salts thereof. Furthermore, in one aspect of the present invention, the bile acid derivative is two or more types selected from the above structural group.

Furthermore, in one aspect of the present invention, in (ii) excipient A, the bile acid derivative is one or more selected from the group consisting of chenodeoxycholic acid, ursodeoxycholic acid, and pharmaceutically acceptable salts thereof. Furthermore, in one aspect of the present invention, the bile acid derivative is two or more types selected from the above structural group.

In another aspect of the present invention, in (ii) excipient A, the bile acid derivative is chenodeoxycholic acid and ursodeoxycholic acid; or a pharmaceutically acceptable salt thereof.

In another aspect of the present invention, in (ii) excipient A, the bile acid derivative includes a tight junction open bile acid derivative.

In another aspect of the present invention, in (ii) excipient A, the bile acid derivative includes a non-tight junction open bile acid derivative.

In one specific aspect of the present invention, in (ii) excipient A, the bile acid derivative includes the tight junction open bile acid derivative A-1 and the non-tight junction open bile acid derivative A-2.

In one aspect of the present invention, excipient B is a compound or a derivative thereof having one or more of CYP450 inhibition, antioxidant effect, or enzyme activity inhibition effect.

In one aspect of the present invention, in the excipient B, at least one compound or derivative thereof having CYP450 inhibition, antioxidant effect, or enzyme activity inhibition effect is selected from the group consisting of CYP450 inhibitory compounds; antioxidant compounds; protease inhibitory compounds; and pharmaceutically acceptable salts thereof. Or, it is two or more types selected from the above group.

In one aspect of the present invention, the CYP450 inhibitory compound is propyl gallate or a pharmaceutically acceptable salt thereof.

In one aspect of the present invention, in excipient B, the antioxidant compound is one or more selected from the group consisting of gallic acid, caffeic acid, lipoic acid, citric acid, acetyl carnitine, acetyl cysteine, glutathione, ascorbyl compounds, tocopheryl compounds, and pharmaceutically acceptable salts thereof. Or, the antioxidant compound is two or more types selected from the above structural group.

In one aspect of the present invention, the ascorbyl compound is one or more selected from the group consisting of ascorbyl palmitate, ascorbyl stearate, and pharmaceutically acceptable salts thereof.

In one aspect of the present invention, the tocopheryl compound is one or more selected from the group consisting of tocopherol, tocopheryl acetate, tocopheryl succinate, and pharmaceutically acceptable salts thereof.

In one aspect of the present invention, the proteolytic enzyme inhibitory compound has an effect of inhibiting enzyme activity in the gastrointestinal tract.

In one aspect of the present invention, the proteolytic enzyme inhibitory compound is one or more selected from the group consisting of propyl gallate, aprotinin, camostat mesylate, gabexate mesylate, soybean trypsin inhibitor (soybean Kunitz trypsin inhibitor, SBTI), soybean trypsin-chymotrypsin inhibitor (soybean Kunitz trypsin-chymotrypsin inhibitor, SBTCI), soybean Bowman-Birk inhibitor, ethylenediaminetetraacetic acid (EDTA), Bacitracin, ovomucoid, citric acid, and pharmaceutically acceptable salts thereof. Or, the antioxidant compound is two or more types selected from the above structural group.

In one aspect of the present invention, the (iii) excipient B is one or more selected from the group consisting of propyl gallate, camostat mesylate, citric acid, soybean trypsin inhibitor, EDTA, and pharmaceutically acceptable salts

thereof. Or, the antioxidant compound is two or more types selected from the above structural group.

In one aspect of the present invention, the (iii) excipient B is one or more selected from the group consisting of propyl gallate, camostat mesylate, and pharmaceutically acceptable salts thereof. Or, it is two or more types selected from the above structural group.

In one specific aspect of the present invention, the (ii) excipient A is one or more selected from the group consisting of chenodeoxycholate, ursodeoxycholate and pharmaceutically acceptable salts thereof; and the (iii) excipient B is one or more selected from the group consisting of propyl gallate, camostat mesylate, and pharmaceutically acceptable salts thereof. Or, each excipient is two or more selected from the above group.

In one aspect of the present invention, the pharmaceutical composition comprises excipient A and excipient B; the weight ratio of excipient A to excipient B is 1 :0.001 to 5.

In one aspect of the present invention, the weight ratio of the (i) large physiologically active substance to (ii) excipient A is 1:1 to 1500.

In one aspect of the present invention, (ii) excipient A comprises two or more bile acid derivatives; the weight ratio of each is 1 to 1500 relative to the weight of the large physiologically active substance.

In one aspect of the present invention, the weight ratio of the (i) large physiologically active substance and (iii) excipient B is 1:0.1 to 300.

In one aspect of the present invention, the pharmaceutical composition is administered orally.

Furthermore, the present invention relates to a method for producing a pharmaceutical composition, including a step of mixing(i) a large physiologically active substance; and (ii) an excipient A comprising a bile acid derivative, or (iii) an excipient B comprising a compound or derivative thereof having CYP450 inhibition, antioxidant effect, or enzyme activity inhibition effect.

In one aspect of the present invention, the weight ratio of the (i) large physiologically active substance to (ii) excipient A is 1:1 to 1500.

In one aspect of the present invention, the weight ratio of the (i) large physiologically active substance to (iii) excipient B is 1:0.1 to 300.

In one aspect of the present invention, in the above manufacturing method, the pharmaceutical composition comprises excipient A and excipient B; the weight ratio of excipient A to excipient B is 1:0.001 to 5.

[Effect of the Invention]

In the present invention, by using two or more excipients, the decomposition of large physiologically active substances can be prevented, and the absorption rate in the body is significantly increased as the intestinal membrane permeation is possible, which has the advantage of having an excellent absorption rate.

[Brief Description of the Drawings]

FIG. 1 is a diagram confirming the cytotoxicity due to bile acid derivatives in Caco-2.

FIG. 2 is a diagram confirming the tight junction openness of bile acid derivatives and propyl gallate in Caco-2.

FIG. 3 is a diagram confirming the Caco-2 cell membrane permeation pathway by bile acid derivatives.

FIG. 4 is a diagram measuring the Caco-2 cell membrane permeability of a composition comprising a large physiologically active substance (compounds 1 and 14) and one type of bile acid.

FIGS. 5 and 6 are diagrams measuring the blood sugar regulation ability of a composition comprising a large physiologically active substance (compound 1), one or more bile acid derivatives, and propyl gallate.

FIGS. 7 and 8 are diagrams measuring the weight loss and appetite suppression effects of a composition comprising a large physiologically active substance (compound 14), one or more bile acid derivatives, and propyl gallate.

FIGS. 9 and 10 are diagrams confirming the weight loss and appetite suppression effects of a composition comprising a large physiologically active substance (compound 15), one or more bile acid derivatives, and propyl gallate.

FIGS. 11 to 13 are diagrams confirming the weight loss and appetite suppressing effects of a composition comprising a large physiologically active substance (compound 17), one or more bile acid derivatives, and propyl gallate.

FIG. 14 is a diagram confirming the blood sugar regulation ability of a composition comprising a large physiologically active substance (compound 18), one or more bile acid derivatives, and propyl gallate.

FIGS. 15 and 16 are diagrams confirming the blood sugar regulation ability of a composition comprising a large physiologically active substance (compound 19), one or more bile acid derivatives, and propyl gallate.

FIG. 17 is a diagram confirming the blood sugar regulation ability of a composition comprising a large physiologically active substance (compounds 20, 21, and 22), one or more bile acid derivatives, and propyl gallate.

[Best Mode for Carrying Out the Invention]

The present invention relates to a pharmaceutical composition, comprising (i) a large physiologically active substance; and (ii) excipient A comprising a bile acid derivative; or (iii) excipient B comprising a compound or a derivative thereof having CYP450 inhibition, antioxidant effect, or gastrointestinal enzyme activity inhibition effect.

[Mode for Carrying Out the Invention]

Hereinafter, implementation examples and examples of the present invention will be described in detail so that those skilled in the art can easily practice the present invention.

However, the present invention may be implemented in various different forms and is not limited to the implementation examples and examples described herein. Throughout the specification of the present invention, when it is said that a part "includes" a certain component, this means that it does not exclude other components but may further include other components, unless specifically stated otherwise.

In the entirety of the specification of the present invention, the terms "combination thereof" and "combination thereof" included in the Markushi format expression refer to one or more mixtures or combinations selected from the group consisting of the constituent elements described in the Markushi format expression, which means that one or more selected from the group consisting of the above components are included.

In the present invention, "derivative" and "analog" mean that part of the structure has been changed due to deletion, substitution, addition, and the like.

In the present invention, "pharmaceutically acceptable" means that the included ingredients do not significantly irritate living organisms and do not inhibit biological activities and properties.

In the present invention, "pharmaceutically acceptable salt" refers to a salt that possesses desirable biological activity without hanning the biological activity and characteristics of humans or animals, but is not limited thereto; it comprises inorganic acid salts (hydrochloric acid, sulfuric acid, phosphoric acid, nitric acid), organic acid salts (acetic acid, oxalic acid, maleic acid, fumaric acid, succinic acid, benzoic acid, ascorbic acid, tannic acid, pamoic acid, alginic acid, triethylamine, cyclohexylamine, pyridine), alkali metal salts (sodium salt, potassium salt), alkaline earth metal salts (calcium salts), ammonium salts, their addition salt forms, and the like.

In the present invention, bile acids are amphipathic molecules and can promote drug permeation through biological membranes. Bile acid or its derivatives are absorbed in a combined form with the large physiologically active substance of the present invention, thereby minimizing the loss of the large physiologically active substance during oral administration and improving the absorption rate in the body. Furthermore, in the present invention, bile acids or their derivatives can be mixed with other excipients to further improve absorption in the body. The absorption rate in the body can be adjusted to permeate at a desired location in the intestine according to the above combination. For example, by inhibiting the decomposition of large physiologically active substances in the stomach, the formulation can reach the small intestine or colon and act.

Furthermore, considering cell stability, cytotoxicity, absorption rate in the body, and the like, bile acid derivatives wherein part of the bile acid is substituted, deleted, or added can be appropriately selected.

The present invention relates to a pharmaceutical composition, comprising (i) a large physiologically active substance; and (ii) an excipient A comprising a bile acid derivative; or (iii) an excipient B comprising a compound or derivative thereof having CYP450 inhibition, antioxidant effect, or enzyme activity inhibition effect.

In the present invention, a large physiologically active substance has a molecular weight known in the art, and specifically refers to a substance that has a size of 1000 Da or more and is active in the human or animal body.

The large physiologically active substance includes polypeptides, proteins, polysaccharides, nucleotides, or analogs thereof, and includes the following examples:

(a) glucagon, GLP-1, GLP-2, GIP, exendin-4, exenatide, semaglutide, liraglutide, insulin, parathyroid hormone, tirzepatide, amylin, pramlintide, lipidated exendin-4, lipidated exenatide, lipidated insulin, lipidated amylin, lipidated GluB, biotinyl Exendin-4, biotinyl exenatide, biotinyl amylin, biotinyl parathyroid hormone, octreotide, leuprolide, goserelin, growth hormone, etanercept, antibodies, antibody fragments, albumin and fragments thereof, galanin, calcitonin, secretin, histone, interferon, erythropoietin, serotonin, rituximab, trastuzumab, uricase, tissue plasminogen activator, thymoglobin, vaccine, transsferrin, fibronectin, antithrombin III, filgrastim, pramlintide acetate, eptifibatide, myosin, actin, dystrophin, antivenin, IgG, IgM, HGH, thyroxine, blood coagulation factor VII, blood coagulation factor VIII, monoclonal antibodies, antigen sections, interleukins, cytokines, TNF, TGF, enzymes, binding proteins, cell receptors, signaling proteins, signaling molecules, their analogs, and their conjugates;

(b) glycogen, labulose, lactose, glucan, chitin, glycogen, cellulose, starch, dextrin, pectin, heparin, chitosan, schizophyllan, starch, araban, fructan, hyaluronic acid, keratan, chondroitin, xylan, analogs thereof, conjugates thereof; and

(c) ribonucleotides including AMP, GMP, UMP, CMP, IMP, XMP, and the like.; deoxyribonucleotides including dAMP, dGMP, dUMP, dCMP, dIMP, dXMP, and the like.; cyclic nucleotides including cAMP, cGMP, c-di-GMP, c-di-AMP, cADP, and the like.; nucleoside diphosphates including ADP, GP, UDP, CDP, dADP, and the like.; oligonucleotides and polynucleotides including combinations thereof; analogues thereof, conjugates thereof, and the like. However, the substances listed above are examples and the large physiologically active substance is not limited thereto.

In the present invention, biotinylated refers to a form wherein part of a polypeptide, protein, polysaccharide, and the like is bound to a biotin moiety, and the biotin moiety refers to a vitamin B, vitamin B complex, or part or all of the vitamin B analogues. Furthermore, in this case, the vitamin B complex refers to a complex form including one or more vitamin B in a form wherein vitamin B is bound to amino acids, fatty acids, and the like.

Furthermore, in the present invention, "lipidated" refers to a form wherein part of a polypeptide, protein, polysaccharide, and the like. is bound to a fatty acid moiety, and fatty acid moiety refers to part or all of carboxylic acid that has a long aliphatic chain that is either saturated or unsaturated. Fatty acid moieties that can be bound include, for example, caprylic acid, lauric acid, palmitic acid, stearic acid, arachidic acid, cerotic acid, which are types of saturated acids, myristoleic acid, palmitoleic acid, oleic acid, linoleic acid, alpha-linolenic acid, and the like, which are types of saturated fatty acids. However, these are examples and the fatty acid moieties are not limited thereto.

Furthermore, in the present invention, biotinylation/lipidation refers to a form that includes both biotinylation and lipidation, and means a form wherein a biotin moiety and a fatty acid moiety are bound to a portion of a polypeptide, protein, polysaccharide, and the like. Furthermore, biotinylation and lipidation may exist in a form that includes both biotinylation and lipidation in one amino acid (for example, amino acid-fatty acid moiety-biotin moiety, amino acid-biotin moiety-fatty acid moiety-biotin moiety, and the like) or may exist in a form respectively including biotinylation and lipidation, at two or more different amino acids.

In one aspect of the present invention, the (i) large physiologically active substance is a polypeptide, protein, polysaccharide, nucleotide, or an analog thereof.

In one aspect of the present invention, the (i) large physiologically active substance is one or more selected from the group consisting of GLP-1, GCG, GIP, insulin, amylin, parathyroid hormone, calcitonin, heparin, human growth hormone, erythropoietin and analogs thereof.

In one aspect of the present invention, the (i) large physiologically active substance is one or more selected from the group consisting of GLP-1, GCG, GIP, insulin, amylin, parathyroid hormone, and analogs thereof.

In one aspect of the present invention, the (i) large physiologically active substance is one or more selected from the group consisting of GLP-1 receptor agonist, GCG receptor agonist, GIP receptor agonist, GLP-1/GCG receptor agonist, GLP-1/GCG/GIP receptor agonist, insulin, insulin receptor agonist, amylin, amylin receptor agonist, parathyroid hormone, parathyroid hormone receptor agonist, biotinylated analogs thereof, lipidated analogs thereof, and biotinylated/lipidated analogs thereof.

In one specific aspect of the present invention, the (i) large physiologically active substance is one or more selected from the group consisting of GLP-1 receptor agonist, GLP-1/GCG receptor agonist, insulin, insulin receptor agonist, amylin, amylin receptor agonist, parathyroid hormone, parathyroid hormone receptor agonist, biotinylated analog thereof, lipidated analog thereof, and biotinylated/lipidated analog thereof.

In one aspect of the present invention, the (i) large physiologically active substance is one or more selected from the group consisting of glucagon, GLP-1 (glucagon-like peptide-1), GLP-2 (glucagon-like peptide-2), GIP (glucose-dependent insulinotropic polypeptide), exendin-4, exenatide, semaglutide, liraglutide, insulin, parathyroid hormone (PTH), tirzepatide, amylin, pramlintide, lipidated Exendin-4, lipidated exenatide, lipidated insulin, lipidated amylin, lipidated GluB, biotinyl Exendin-4, biotinyl exenatide, biotinyl amylin, biotinyl-PTH, octreotide, goserelin, leuprolide, growth hormone, etanercept, and the analogs thereof.

Furthermore, in one aspect of the present invention, the (i) large physiologically active substance is one or more selected from the group consisting of exendin-4, exenatide, semaglutide, Insulin, parathyroid hormone (PTH), amylin, analogs thereof, biotinylated analogs thereof, lipidated analogs thereof, and biotinylated/lipidated analogs thereof.

The biotinylation can be achieved through amino acid, polypeptide, alkylene, amine, or polyamidoamine structures. In this case, the amino acid includes lysine, 5-hydroxylysine, 4-oxalysine, 4-thialysine, 4-selenalysine, 4-thiahomolysine, 5,5-dimethyllysine, 5,5-difluorolysine, trans-4-dihydrolysine, 2,6-diamino-4-hexynoic acid, Cis-4-dihydrolysine, 6-N-methyllysine, diaminopimelic acid, ornithine, 3-methylornithine, $\alpha$-methylornithine, citrulline, homocitrulline, arginine, aspartate, asparagine, glutamate, glutamine, histidine, ornithine, proline, serine, or threonine; these are examples and the amino acid is not limited thereto.

Biotinylation can be achieved by substituting or inserting part of the polypeptide; for example, one or more amino acids in the inactive region of the amino acid sequence shown in SEQ ID NO: 1 may be replaced or inserted by a lysine amino acid. Furthermore, while not limited thereto, for example, one or more of the amino acids in the inactive region of the amino acid sequence shown in SEQ ID NO: 1 may be substituted by 2-aminoisobutyric acid (Aib), and lysine amino acid may be inserted. Furthermore, for example, any one or more amino acids in the inactive portion of the

amino acid sequence may be substituted by pyroglutamic acid (pyr).

In one aspect of the present invention, the biotinylation may be biotinylation in which the following compound is bound:

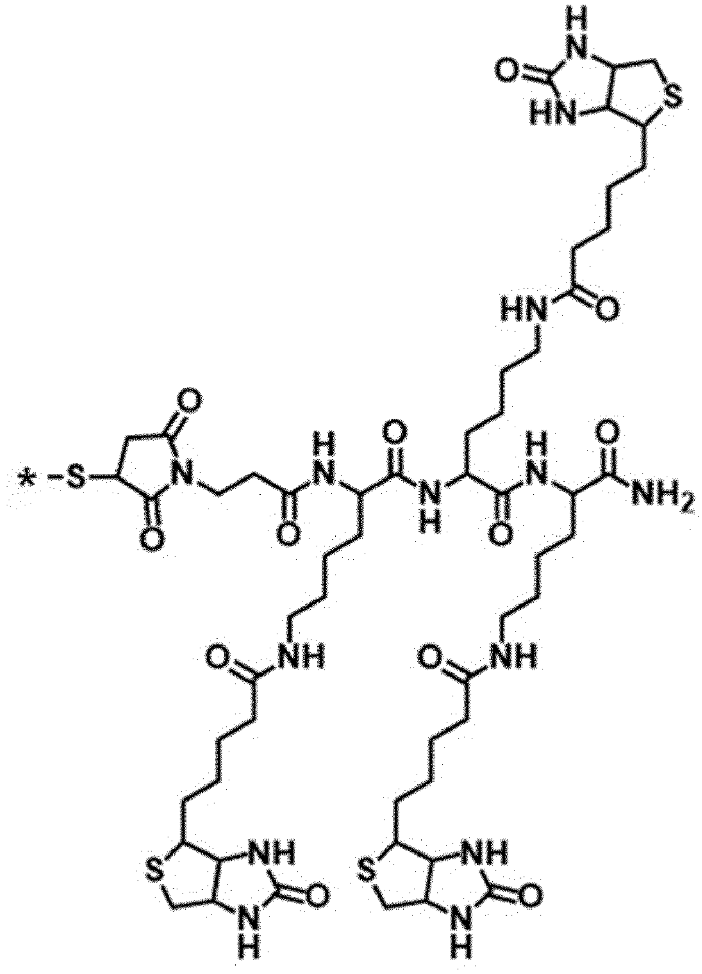

,

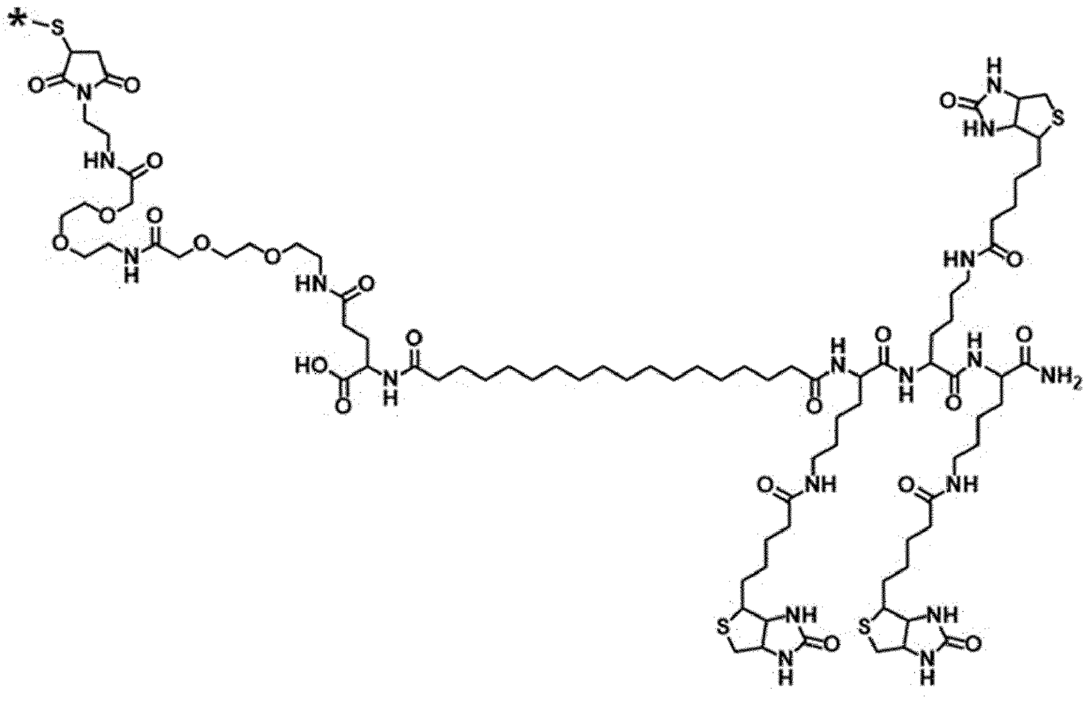

,

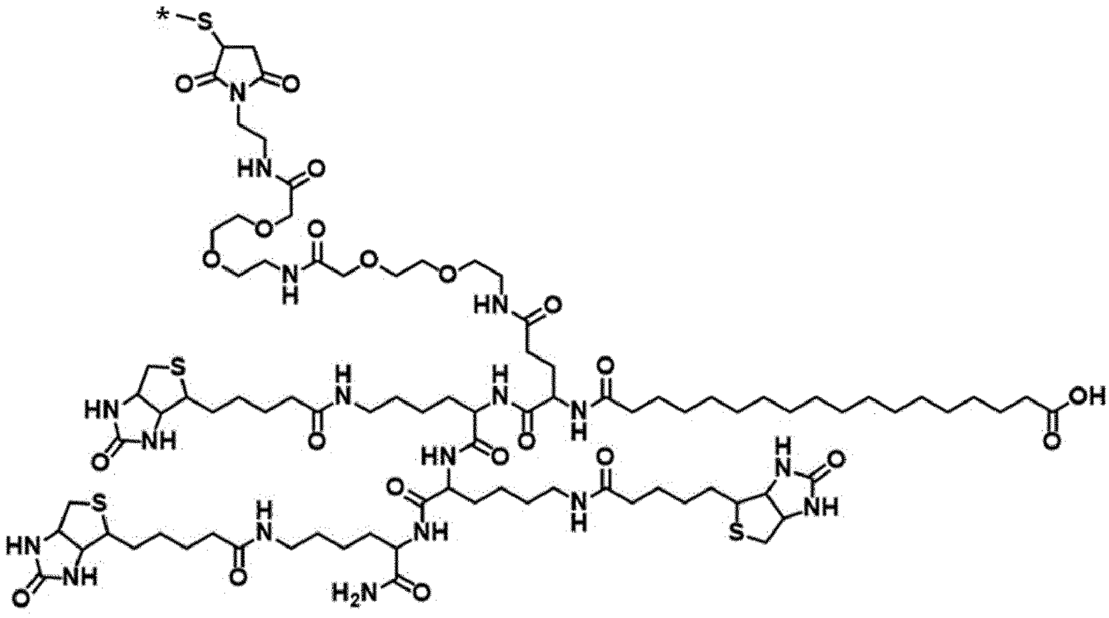

,

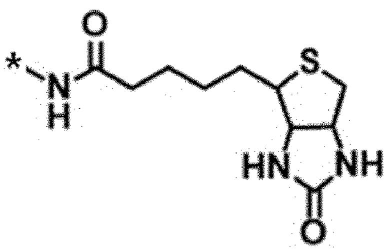

, or

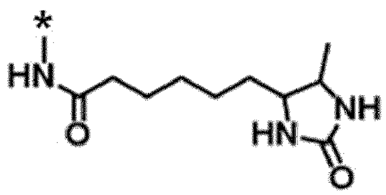

,

In this case, * is the part bound to the amino acid.

Furthermore, in one aspect of the present invention, the lipidation may be lipidation in a state wherein the following compounds are bound:

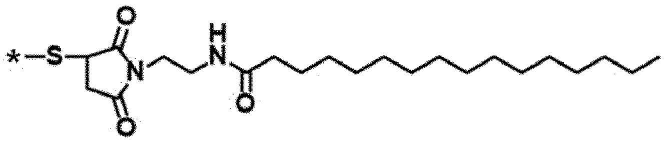

,

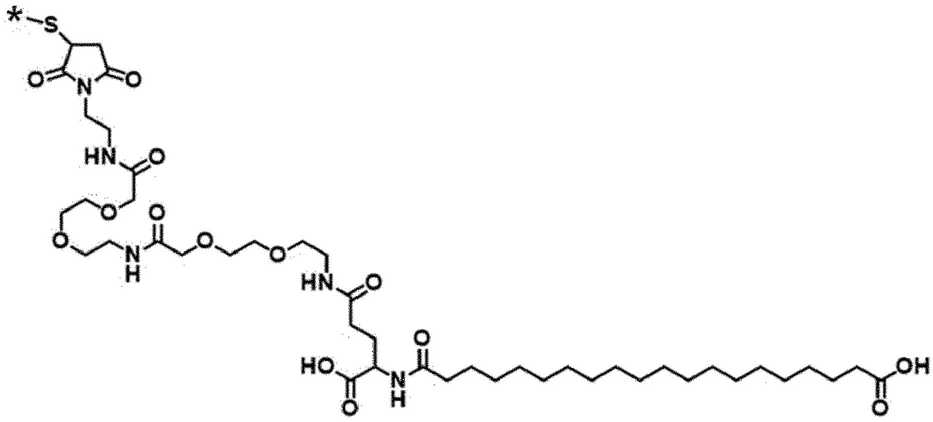

.

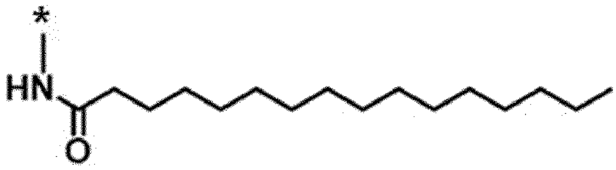

,

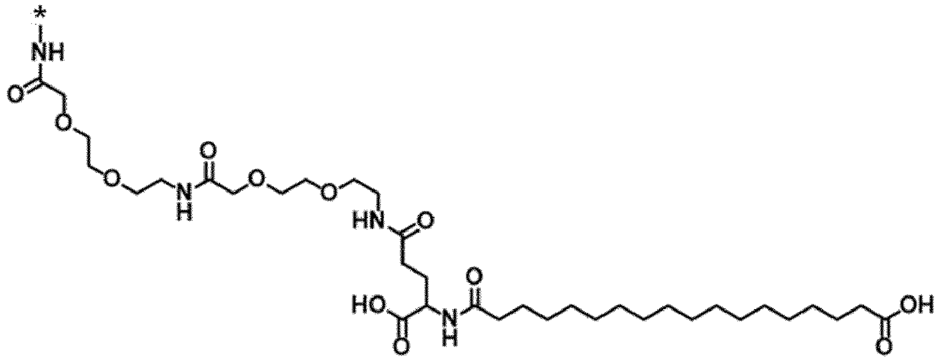

,

or

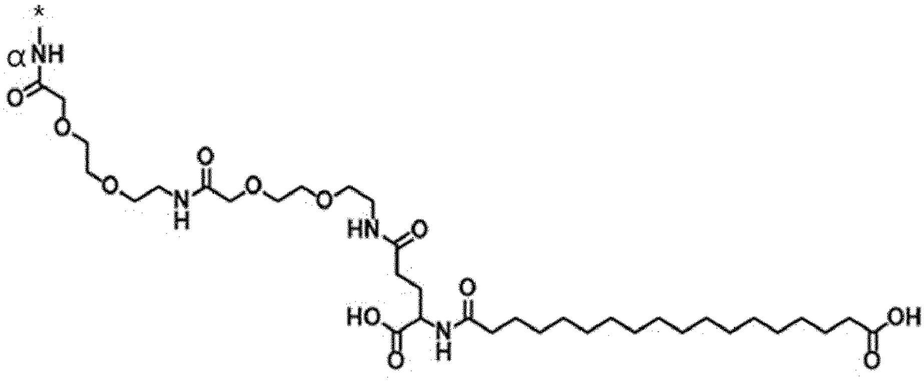

,

In this case, * is the part bound to the amino acid.

In one specific aspect of the present invention, the (i) large physiologically active substance is selected from the group consisting of SEQ ID NOs: 1 to 9 or SEQ ID NOs: 13 to 17. Furthermore, in one specific aspect of the present invention, the (i) large physiologically active substance may be a protein having the amino acid sequences of SEQ ID NOs: 10 and 11 or a protein having the amino acid sequences of SEQ ID NOs: 12 and 11.

Furthermore, in one specific aspect of the present invention, the (i) large physiologically active substance may be a polypeptide wherein a biotin moiety or a fatty acid moiety is bound to one or more amino acids of the protein composed of the above sequence number.

In the present invention, excipients A and B assist, promote and enhance the absorption of the large physiologically active substance in vivo, thereby increasing the absorption rate.

In one aspect of the present invention, the (ii) excipient A comprising a bile acid derivative and the (iii) excipient B comprising a compound having a CYP450 inhibition, antioxidant effect, or enzyme activity inhibition effect may each comprise one or more types. In the present invention, the type and number of the excipients A and the excipient B may vary depending on the type of large physiologically active substance.

In one aspect of the present invention, in the excipients A and B, the excipient includes a tight junction open compound or a non-tight junction open compound.

In the present invention, the tight junction open above refers to a type of opening of cell junctions, and in particular, refers to an action mechanism that is able to promote the absorption of macro active molecules by opening the tight junctions of epithelial cells.

In the present invention, the non-tight junction openness (non-tight junction open) refers to all mechanisms that can improve oral absorption rate other than the tight junction open mechanism; for example, this comprises mechanisms such as the opening of junctions other than tight junctions and promoting binding to intestinal receptors, such as decreased decomposition in the gastrointestinal tract, improved gastrointestinal permeability, adhering junctions, desmosomes, hemidesmosomes, gap junctions, and the like in the intestines.

In one aspect of the present invention, in the excipients A and B, the excipients includes one or more selected from tight junction open bile acid derivatives; non-tight junction open bile acid derivatives or non-tight junction open compounds.

In one aspect of the present invention, the tight junction open bile acid derivative may be chenodeoxycholic acid, deoxycholic acid, or a pharmaceutically acceptable salt thereof.

In one aspect of the present invention, the non-tight junction open bile acid derivative may be ursodeoxycholic acid or a pharmaceutically acceptable salt thereof.

In one aspect of the present invention, the non-tight junction open compound may be propyl gallate or a pharmaceutically acceptable salt thereof.

Excipient A comprises a bile acid derivative; here, bile acid derivatives comprise, for example, glycocholic acid, glycocholicchenodeoxycholic acid, taurocholic acid, deoxycholic acid, cholic acid, chenodeoxycholic acid, ursodeoxycholic acid, lithocholic acid, their analogs, conjugates, and the like.

In one aspect of the present invention, in the (ii) excipient A, the bile acid derivative is one or more selected from the group consisting of glycocholic acid, glycocholicchenodeoxycholic acid, taurocholic acid, deoxycholic acid, taurodeoxycholic acid, cholic acid, chenodeoxycholic acid, ursodeoxycholic acid, lithocholic acid, dehydrocholic acid, and pharmaceutically acceptable salts thereof.

In one aspect of the present invention, in the (ii) excipient A, the bile acid derivative is two or more types selected from the group consisting of glycocholic acid, glycocholicchenodeoxycholic acid, taurocholic acid, deoxycholic acid, taurodeoxycholic acid, cholic acid, chenodeoxycholic acid, ursodeoxycholic acid, lithocholic acid, dehydrocholic acid, and the pharmaceutically acceptable salts thereof.

In one aspect of the present invention, in the (ii) excipient A, the bile acid derivative is one or more selected from the group consisting of glycocholic acid, taurocholic acid, deoxycholic acid, cholic acid, chenodeoxycholic acid, ursodeoxycholic acid, and pharmaceutically acceptable salts thereof. Or, it is two or more types selected from the above structural group.

Furthermore, in one aspect of the present invention, in the (ii) excipient A, the bile acid derivative is one or more selected from the group consisting of chenodeoxycholic acid, ursodeoxycholic acid, and pharmaceutically acceptable salts thereof. Or, it is two or more types selected from the above structural group.

Furthermore, in one aspect of the present invention, in the (ii) excipient A, the bile acid derivative comprises chenodeoxycholic acid and ursodeoxycholic acid, or a pharmaceutically acceptable salt thereof.

Compounds that have CYP450 inhibition, antioxidant effect, or enzyme activity inhibition effect in excipient B include:

for example, polyphenol compounds including phenolic acids such as coumaric acid, caffeic acid, ferulic acid, sinapinic acid, and gallic acid, flavonoids such as quercetin, myricetin, catechin, epicatechin, epigallocatechin, apigenin, and luteolin, stilbenes such as resveratrol and pterostilbene, and lignans such as sesamin, sesamolin, nordihydroguaiaretic acid, gomisin A, and secoisolariciresinol;

carnitine compounds such as L-camitine, D-carnitine, acetyl L-carnitine, acetyl D-carnitine, propionyl carnitine, and carnitine hydrochloride;

amino acid moieties including at least one cysteine, cystine, or methionine; sulfur-containing compounds such as cysteine, cystine, methionine, adenosylmethionine, glutathione, glutathione disulfide, taurine, thiamine, biotin, alpha-lipoic acid, dihydro-alpha-lipoic acid, and the like;

proteolytic enzyme inhibitors such as plant-derived trypsin inhibitor, soybean trypsin inhibitor, Bowman-Birk inhibitor, corn protease inhibitor, animal-derived trypsin inhibitor, chymostatin, nafamostat mesylate, camostat mesylate, gabexate mesylate, aprotinin, antipain, benzamidine, leupeptin, pepstatin, phosphoramidon, 4-(2-aminoethyl)benzenesulfonyl fluoride hydrochloride (AEBSF) , tosyl-L-lysyl-chloromethane hydrochloride (TLCK), tosyl-L-phenylalanyl fluoromethyl ketone (TPCK), aminophenylmethanesulfonyl fluoride hydrochloride (APMSF), diisopropylfluorophosphate ( DFP), phenylmethanesulfonylfluoride (PMSF), ovomucoid, sepimostat, amastatin, bestatin, diprotin A, bacitracin, puromycin, citric acid, trisodium citrate and dextrose; and

chelating agents such as ethylenediaminetetraacetic acid (EDTA), chitosan-EDTA conjugate, citrate, EGTA, diethylenetriamine pentaacetic acid (DTPA), and BAPTA.

Furthermore, the excipient B may additionally include pharmaceutically acceptable compounds, such as, for example, vitamin C, vitamin E, alpha-tocopherol, malic acid, fumaric acid, ascorbic acid, butylated hydroxyanisole, butylated hydroxy toluene, sodium phosphate, calcium phosphate, potassium phosphate, galactose, glucose, maltose, and the like.

In one aspect of the present invention, the excipient B is a compound or a derivative thereof having one or more of CYP450 inhibition, antioxidant effect, or enzyme activity inhibition effect.

In one aspect of the present invention, in the excipient B, the compound or derivative thereof having the CYP450 inhibition, antioxidant effect, or enzyme activity inhibition effect is one or more selected from the group consisting of CYP450 inhibitory compounds, antioxidant compounds, proteolytic enzyme inhibitory compounds, and pharmaceutically acceptable salts thereof. Or, the antioxidant compound is two or more types selected from the above structural group.

In one aspect of the present invention, the CYP450 inhibitory compound is propyl gallate or a pharmaceutically acceptable salt thereof.

In one aspect of the present invention, the antioxidant compound is one or more selected from the group consisting of gallic acid, caffeic acid, lipoic acid, citric acid, acetyl carnitine, acetyl cysteine, glutathione, ascorbyl compound, tocopherol compound, and pharmaceutically acceptable salts thereof. Or, the antioxidant compound is two or more types selected from the above structural group.

In one aspect of the present invention, the ascorbyl compound is one or more selected from the group consisting of ascorbyl palmitate, ascorbyl stearate, and pharmaceutically acceptable salts thereof.

In one aspect of the present invention, the tocopheryl compound is one or more selected from the group consisting of tocopherol, tocopheryl acetate, tocopheryl succinate, and pharmaceutically acceptable salts thereof.

Furthermore, in one aspect of the present invention, the proteolytic enzyme inhibitory compound has an effect of inhibiting enzyme activity in the gastrointestinal tract.

In one aspect of the present invention, the proteolytic enzyme inhibitory compound is one or more selected from the group consisting of propyl gallate, aprotinin, camostat mesylate, gabexate mesylate, soybean trypsin inhibitor (soybean Kunitz trypsin inhibitor, SBTI), soybean trypsin-chymotrypsin inhibitor (soybean Kunitz trypsin-chymotrypsin inhibitor, SBTCI), soybean Bowman-Birk inhibitor, ethylenediaminetetraacetic acid (EDTA), Bacitracin, ovomucoid, citric acid, and pharmaceutically acceptable salts thereof. Or, the antioxidant compound is two or more types selected from

the above structural group.

Furthermore, in one aspect of the present invention, the proteolytic enzyme inhibitory compound is one or more selected from the group consisting of propyl gallate, camostat mesylate, soy trypsin inhibitor, citric acid, EDTA, and pharmaceutically acceptable salts thereof. Or, the antioxidant compound is two or more types selected from the above structural group.

In one aspect of the present invention, the excipient B is one or more selected from the group consisting of propyl gallate, camostat mesylate, citric acid, soybean trypsin inhibitor, EDTA, and pharmaceutically acceptable salts thereof. Or, the antioxidant compound is two or more types selected from the above structural group.

In one aspect of the present invention, the excipient B is one or more selected from the group consisting of propyl gallate, camostat mesylate, and pharmaceutically acceptable salts thereof. Or, it is two or more types selected from the above structural group.

In one specific aspect of the present invention, the bile acid derivative is one or more selected from the group consisting of chenodeoxycholate, ursodeoxycholate and pharmaceutically acceptable salts thereof, and the excipient B is propyl gallate, camostat mesylate, citric acid, soybean trypsin inhibitor, EDTA, and pharmaceutically acceptable salts thereof. Or, each excipient is two or more types selected from the above group.

In one specific aspect of the present invention, the excipient A is chenodeoxycholate and ursodeoxycholate, or a pharmaceutically acceptable salt thereof; and the excipient B is propyl gallate; propyl gallate and camostat mesylate, or a pharmaceutically acceptable salt thereof.

In general, large physiologically active substances are classified as BCS (Biopharmaceutical Classification System) class 3, which has a restriction in the absorption region in the gastrointestinal tract due to the high water solubility thereof, and there is a restriction in the use thereof as pharmaceutical compositions. However, due to the large physiologically active substance according to one embodiment of the present invention being used together with one type of the excipient A and one type of the excipient B; 2 types of the excipient A and 1 type of the excipient B; or two types of the excipient A and two types of the excipient B, intestinal membrane permeability may be increased.

In one aspect of the present invention, the pharmaceutical composition comprises excipient A and excipient B; the weight ratio of excipient A to excipient B is 1:0.001 to 5. Specifically, the weight ratio is 1:0.002 to 4.9, 1:0.003 to 4.8, 1:0.004 to 4.7, and 1:0.005 to 4.6. More specifically, the weight ratio of the excipient A to the excipient B is 1 :0.005 to 4.5.

In one aspect of the present invention, the pharmaceutical composition comprises an excipient A and an excipient B, and in this case, the weight ratio of the large physiologically active substance to the excipient is 1:1 to 2000. In this case, the excipient is the weight sum of the excipient A and the excipient B. Specifically, the weight ratio of the large physiologically active substance and the excipient is 1:2 to 1900, 1:3 to 1850, 1:4 to 1800, and 1:5 to 1800. More specifically, the weight ratio between the large physiologically active substance and the excipient is 1:5 to 1750.

In one aspect of the present invention, (ii) excipient A comprises two or more bile acid derivatives; the weight ratio of each is 1 to 1500 relative to the weight of the large physiologically active substance. Furthermore, in one aspect of the present invention, the weight ratio of the (i) large physiologically active substance and the (iii) excipient B is 1:0.1 to 300.

In one aspect of the present invention, the excipient A may comprise, as a bile acid or derivative thereof, one or more selected from the group consisting of glycocholic acid, taurocholic acid, deoxycholic acid, deoxycholic acid, cholic acid, chenodeoxycholic acid, ursodeoxycholic acid and pharmaceutically acceptable salts thereof; the weight thereof may be from 0.1 mg/kg to 500 mg/kg, respectively. More specifically, the weight may be 0.1 mg/kg to 300 mg/kg, 0.2 mg/kg to 100 mg/kg, 0.2 mg/kg to 50 mg/kg, 0.2 mg/kg to 30 mg/kg, 0.2 mg/kg to 10 mg/kg, 0.3 mg/kg to 10 mg/kg, 0.3 mg/kg to 8 mg/kg, 0.4 mg/kg to 8 mg/kg, 0.4 mg/kg to 7 mg/kg, and 0.4 mg/kg to 6 mg/kg.

Specifically, it comprises chenodeoxycholate and ursodeoxycholate, and the weight of each may be 0.1 mg/kg to 500 mg/kg. More specifically, the weight may be 0.1 mg/kg to 300 mg/kg, 0.2 mg/kg to 100 mg/kg, 0.2 mg/kg to 50 mg/kg, 0.2 mg/kg to 30 mg/kg, 0.2 mg/kg to 10 mg/kg, 0.3 mg/kg to 10 mg/kg, 0.3 mg/kg to 8 mg/kg, 0.4 mg/kg to 8 mg/kg, 0.4 mg/kg to 7 mg/kg, and 0.4 mg/kg to 6 mg/kg.

In one aspect of the present invention, the excipient B comprises propyl gallate, and the weight thereof may be 0.01 mg/kg to 500 mg/kg. More specifically, the weight may be 0.01 mg/kg to 100 mg/kg, 0.01 mg/kg to 50 mg/kg, 0.01 mg/kg to 30 mg/kg, 0.01 mg/kg to 10 mg/kg, 0.01 mg/kg to 8 mg/kg, 0.01 mg/kg to 7 mg/kg, 0.01 mg/kg to 6 mg/kg, and 0.02 mg/kg to 6 mg/kg.

In one aspect of the present invention, the excipient B comprises EDTA, and the weight thereof may be 0.01 mg/kg to 500 mg/kg. More specifically, the weight may be 0.01 mg/kg to 100 mg/kg, 0.01 mg/kg to 50 mg/kg, 0.01 mg/kg to 30 mg/kg, 0.01 mg/kg to 10 mg/kg, 0.01 mg/kg to 8 mg/kg, 0.01 mg/kg to 7 mg/kg, 0.01 mg/kg to 6 mg/kg, and 0.02 mg/kg to 6 mg/kg.

In one aspect of the present invention, the excipient B comprises camostat mesylate, and the weight thereof may be 0.001 mg/kg to 10 mg/kg. More specifically, the weight may be 0.001 mg/kg to 5 mg/kg, 0.002 mg/kg to 5 mg/kg, 0.003 mg/kg to 3 mg/kg, 0.005 mg/kg to 2 mg/kg, or 0.005 mg/kg to 1 mg/kg.

In one aspect of the present invention, the excipient B comprises soy Kunitz trypsin inhibitor (SBTI), and the weight thereof may be 0.001 mg/kg to 10 mg/kg. More specifically, the weight may be 0.001 mg/kg to 5 mg/kg, 0.002 mg/kg to 5 mg/kg, 0.003 mg/kg to 3 mg/kg, and 0.005 mg/kg to 1 mg/kg.

In one aspect of the present invention, the excipient B comprises citric acid, and the weight thereof may be 0.01 mg/kg to 100 mg/kg. More specifically, the weight may be 0.01 mg/kg to 50 mg/kg, 0.05 mg/kg to 50 mg/kg, 0.05 mg/kg to 30 mg/kg, 0.07 mg/kg to 30 mg/kg, and 0.07 mg/kg, to 20 mg/kg.

In one aspect of the present invention, the pharmaceutical composition is administered orally.

Specifically, compared to the case wherein only large physiologically active substances are present, the oral absorption rate may be improved by 0.5% or more, 0.6% or more, 0.7% or more, 0.8% or more, 0.9% or more, 1% or more, 1.1% or more, 1.2% or more, 1.3% or more, 1.4% or more, 1.5% or more, 1.6% or more, 1.7% or more, 1.8% or more, 1.9% or more, and 2% or more.

In the present invention, the pharmaceutical composition may have therapeutic or preventive effects depending on the type of the large physiologically active substance. In the pharmaceutical composition according to an example of the present invention, the large physiologically active substance may be a substance used to prevent or treat diabetes, prevent or treat obesity, prevent or treat osteoporosis, prevent or treat fatty liver disease, prevent or treat irritable bowel syndrome, and prevent or treat neurodegenerative diseases. However, these are examples and the fatty acid moieties are not limited thereto.

In one aspect of the present invention, a pharmaceutical composition for preventing or treating diabetes, obesity, fatty liver disease, intestinal disease, and neurodegenerative disease containing the pharmaceutical composition may be provided.

The pharmaceutical composition of the present invention may further comprise excipients in addition to the excipient A and the excipient B. In this case, while not limited thereto, the excipients may comprise stabilizers, surfactants, plasticizers, lubricants, solubilizers, buffers, sweeteners, substrates, adsorbents, flavor correcting agent, binders, suspending agents, antioxidants, brighteners, coating agents, flavoring agents, flavorings, wetting agents, moisture regulators, anti-foaming agents, masticating agents, freshening agents, colorants, sugar coating agents, isotonic agents, pH adjusters, softeners, emulsifiers, adhesives, adhesion enhancers, viscous agents, thickening agents, foaming agents, excipients, dispersants, propellants, disintegrants, disintegration aids, fragrances, dehumidifiers, antiseptics, preservatives, analgesics, solvents, solubilizers, solubilizing agents, fluidizers, and the like.

In the present invention, the pharmaceutical composition may further comprise starch, calcium carbonate, sucrose or lactose, gelatin, and the like for solid preparations; and suspensions, solutions for internal use, emulsions, syrups, and the like for liquid preparations; it may additionally comprise lubricants, humectants, sweeteners, fragrances, preservatives, and the like. Additionally, calcium or vitamin D3 can be added to improve efficacy as a treatment for proliferative diseases or autoimmune diseases. In the present invention, pharmaceutical preparations for oral administration may exist in dosage unit forms such as, for example, dragees, tablets, pills, powders, granules or capsules, and in ampoules. These are prepared using a publicly known method, for example, using conventional mixing, granulating, confectioning, dissolving or lyophilizing methods. For example, pharmaceutical preparations for oral administration can be prepared by mixing the large physiologically active substance with a solid carrier, granulating the mixture, adding appropriate additives as necessary, and then formulating the mixture or granules in the form of tablets or dragees.

In one aspect of the present invention, the pharmaceutical composition is a solid preparation. In one specific aspect of the present invention, the pharmaceutical composition is granules or tablets.

The dosage of the pharmaceutical composition according to one embodiment of the present invention may vary depending on the patient's weight, age, gender, health status, diet, administration time, administration method, excretion rate, and severity of the disease, but, in general, the administered can be performed once a day or in divided doses within the effective daily dosage range. Furthermore, an effective dose may be administered by administration of several times in 1 to 2 weeks.

Furthermore, the present invention provides a method for producing a pharmaceutical composition, including a step of mixing (i) a large physiologically active substance; and (ii) excipient A comprising a bile acid derivative; or (iii) excipient B comprising a compound or a derivative thereof having CYP450 inhibition, antioxidant effect, or gastrointestinal enzyme activity inhibition effect.

Furthermore, the present invention provides a method for producing a pharmaceutical composition including: a step of producing granules by including a step of mixing (i) a large physiologically active substance; and (ii) excipient A comprising a bile acid derivative; or (iii) excipient B comprising a compound or a derivative thereof having CYP450 inhibition, antioxidant effect, or gastrointestinal enzyme activity inhibition effect; and a step of producing tablets using a tablet press and then coating them with a coating machine.

In the present invention, the mixing step can be appropriately adjusted depending on the characteristics of the large physiologically active substance and excipients. While not limited to these, but for example, simple mixing, wet granulation, and dry granulation methods can be performed.

The specific types, weight ratios, and usage amounts of the excipient A and the excipient B are as above.

Furthermore, the present invention relates to a prevention or treatment method including the pharmaceutical composition.

Furthermore, the present invention relates to a method including the pharmaceutical composition for preventing or treating diabetes, obesity, fatty liver disease, intestinal disease, and neurodegenerative disease.

Hereinafter, the present invention will be described in detail using examples and experimental examples. However, the following examples and experimental examples are merely illustrating the present invention, and the content of the present invention is not limited to the following examples and experimental examples.

**<Embodiment 1: Combination of large physiologically active substance and excipient>**

**Large physiologically active substance**

Large physiologically active substances (Macromolecules) were used as shown in Table 1 below. The types and usage amounts of each large physiologically active substance are shown in Table 1. In this case, exendin-4 derivatives, lipidated insulin, lipidated amylin, and the like can be manufactured according to the method disclosed in Korean Patent Application Nos. 10-2019-0064370, 10-2020-0163362, 10-2020-0163363, and 10-2020-0065484.

[Table 1]

| Compound | SEQ No. | Series | SEQ |
|---|---|---|---|
| 1 | 1 | GLP-1RA | HGEGTFTSDLSKQMEEEAVRLFIEWLKNG GPSSGAPPPS |
| 2 | 2 | GLP-1RA | HGEGTFTSDLSKQMEEEAVRLFIEWLKNG GPSSGAPPPSC |
| 3 | 3 | GLP-1RA | HGEGTFTSDLSKQMEEEAVRLFIEWLKNG GPSSGAPPPSK |
| 4 | 4 | GLP-1RA | H-Aib-GEGTFTSDLSKQMEEEAVRLFIEWLKNGG PSSGAPPPS |
| 5 | 2 | GLP-1RA | HGEGTFTSDLSKQMEEEA-VRLFIEWLKNG GPSSGAPPPSC<br>- C40 : Cys-EG2-Glu-C18-B3 (C") |
| 6 | 2 | GLP-1RA | HGEGTFTSDLSKQMEEEA-VRLFIEWLKNG GPSSGAPPPSC<br>- C40 : Cys-B3 ($C^{\#}$) |
| 7 | 2 | GLP-1RA | HGEGTFTSDLSKQMEEEA-VRLFIEWLKNG GPSSGAPPPSC<br>- K27 : Lys-C16 (K*)<br>- C40 : Cys-B3 (C*) |
| 8 | 2 | GLP-1RA | HGEGTFTSDLSKQMEEEA-VRLFIEWLKNG GPSSGAPPPSC<br>- K12 : Lys-B1 ($K^{\#}$)<br>- K27 : Lys-B1 ($K^{\#}$)<br>- C40 : CyS-C16 (C*) |
| 9 | 2 | GLP-1RA | HGEGTFTSDLSKQMEEEA-VRLFIEWLKNG GPSSGAPPPSC<br>- K27 : Lys-EG2-Glu-C18 (K")<br>- C40 : Cys-B3 ($C^{\#}$) |
| 10 | 2 | GLP-1RA | HGEGTFTSDLSKQMEEEA-VRLFIEWLKNG GPSSGAPPPSC<br>- K12 : Lys-B1 ($K^{\#}$)<br>- K27 : Lys-B1 ($K^{\#}$)<br>- C40 : Cys-EG2-Glu-C18 (C") |

(continued)

| Compound | SEQ No. | Series | SEQ |
|---|---|---|---|
| 11 | 2 | GLP-1RA | HGEGTFTSDLSKQMEEEA-VRLFIEWLKNG GPSSGAPPPSC<br>- C40 : Cys-EG2-Glu-C18-B3 (C##) |
| 12 | 2 | GLP-1RA | HGEGTFTSDLSKQMEEEA-VRLFIEWLKNG GPSSGAPPPSC<br>- C40 : Cys-EG2-Glu-B3-C18 (C###) |
| 13 | 2 | GLP-1RA | HGEGTFTSDLSKQMEEEA-VRLFIEWLKNG GPSSGAPPPSC<br>- K12 : Lys-Desthiobiotin (K##)<br>- K27 : Lys-Desthiobiotin (K*)<br>- C40 : Cys-EG2-Glu-C18 (C") |
| 14 | 5 | GLP-1RA | H-Aib-EGTFTSDLSKQMEEEA VRLFIEWLKNGGP SSGAPPPSK<br>- K12 : Lys-B1 (K*)<br>- K27 : Lys-B1 (K*)<br>- K40 : Lys-EG2-Glu-C18 (K") |
| 15 | 6 | GLP-1 RA | H-Aib-EGTFTSDVSSYLEGQAAKEFI-AWLVRGRG<br>- K20 : Lys-EG2-Glu-C18 (K") |
| 16 | 7 | GLP-1/ GCGRA | H-Aib-QGTFTSDYSKYLDEQAAKEFVQWLMNTC |
| 17 | 8 | GLP-1/ GCGRA | H-Aib-QGTFTSDYSKYLDEQAAKE-FVQWLMNTK<br>( Lactam ) between **E16** and **K2C**)<br>- K12 : Lys-B1 (K*)<br>- K30 : Lys-EG2-Glu-C18 (K") |
| 18 | 9 | GLP-1/ GCGRA | Y-Aib-QGTFTSDYSKLLDYMMQRD-FVQWLLEGG PSSGAPPPSK<br>- K12 : Lys-EG2-Glu-C18 (K")<br>- K40 : Lys-B1 (K*) |
| 19 | 10/11 | Insulin | A chain: GIVEQCCTSICSLYQLENYCN (SEQ No. 10)<br>B chain: FVNQHLCGSHL VEAL YL VCGERGF-FYTPKT<br>(SEQ No. 11) |
| 20 | 12/11 | Insulin | A chain: GIVEQCCTSICSLEQLENYCN (SEQ No. 12)<br>B chain: FVNQHLCGSHLVEALYLVCGERGF-FYTPKT<br>(SEQ No. 11) |

(continued)

| Compound | SEQ No. | Series | SEQ |
|---|---|---|---|
| 21 | 12/11 | insulin | A chain: GIVEQCCTSICSLEQLENYCN (SEQ No. 12)<br>B chain: FVNQHLCGSHLVEALYLVCGERGF-FYTPKT (SEQ No. 11)<br>- K29 : Lys-B1 (K*) |
| 22 | 12/11 | Insulin | A chain: GIVEQCCTSICSLEQLENYCN (SEQ No. 12)<br>B chain: FVNQHLCGSHLVEALYLVCGERGF-FYTPKT (SEQ No. 11)<br>- F1 : Phe-EG2-Glu-C18 (F")<br>- K29 : Lys-B1 (K*) |
| 23 | 13 | PTH | SVSEIQLMHNLGKHLNSMERVEWLRKKLQ DVHNF |
| 24 | 13 | PTH | SVSEIQLMHNLGKHLNSMERVEWL-RKKLO DVHNF<br>- K13 : Lys-B1 ($K^{\#}$)<br>- K26 : Lys-B1 ($K^{\#}$)<br>- K27 : Lys-B1 ($K^{\#}$) |
| 25 | 14 | Amylin | KCNTATCATQRLANFLVHSSNNFGPILPPT NVGSNTY |
| 26 | 14 | Amylin | KCNTATCATQRLANFLVHSSNNFG-PILPPT NVGSNTY<br>- K1 : Lys-B1 (K*) |
| 27 | 14 | Amylin | KCNTATCATQRLANFLVHSSNNFG-PILPPT NVGSNTY<br>- K1 : $\alpha$Lys -EG2-Glu-C18 ("K)<br>- K1 : Lys-B1 ($K^{\#}$) |
| 28 | 15 | Amylin | KCNTATCATQRLADFLRHSSPNFGAIPSST NVGSRTY |
| 29 | 15 | Amylin | KCNTATCATQRLADFLRHSSPNF-GAIPSST NVGSRTY<br>- K1 : Lys-B1 ($K^{\#}$) |
| 30 | 15 | Amylin | KCNTATCATQRLADFLRHSSPNF-GAIPSST NVGSRTY<br>- K1 : $\alpha$Lys-EG2-Glu-C18 ("K)<br>- K1 : Lys-B1 ($K^{\#}$) |
| 31 | 16 | Amylin | KCNTATCATQRLADFLRHSSNNFGAIPSST NVGSRTY |
| 32 | 16 | Amylin | KCNTATCATQRLADFLRHSSNNF-GAIPSST NVGSRTY<br>- K1 : Lys-B1 ($K^{\#}$) |

(continued)

| Compound | SEQ No. | Series | SEQ |
|---|---|---|---|
| 33 | 16 | Amylin | KCNTATCATQRLADFLRHSSNNF-GAIPSST NVGSRTY<br>- K1 : αLys-EG2-Glu-C18 ("K)<br>- K1 : Lys-B1 ($K^{\#}$) |
| 34 | 17 | Amylin | RCNTATCATQRLADFLRHSSNNFGAIPSST NVGSKTY |
| 35 | 17 | Amylin | RCNTATCATORLADFLRHSSNNF-GAIPSST NVGSKTY<br>- K35 : Lys-B1 ($K^{\#}$) |

(continued)

| Compound | SEQ No. | Series | SEQ |
|---|---|---|---|
| 36 | 17 | Amylin | RCNTATCATQRLADFLRHSSNNF-GAIPSST NVGSKTY<br>- R1 : αArg-EG2-Glu-C18 ("R)<br>- K35 : Lys-B1 (K#) |

* Structural formula

C$^{*}$ (Cys-C16)

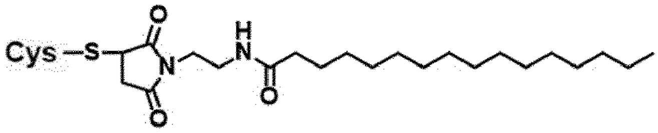

C$^{**}$ (Cys-EG2-Glu-C18)

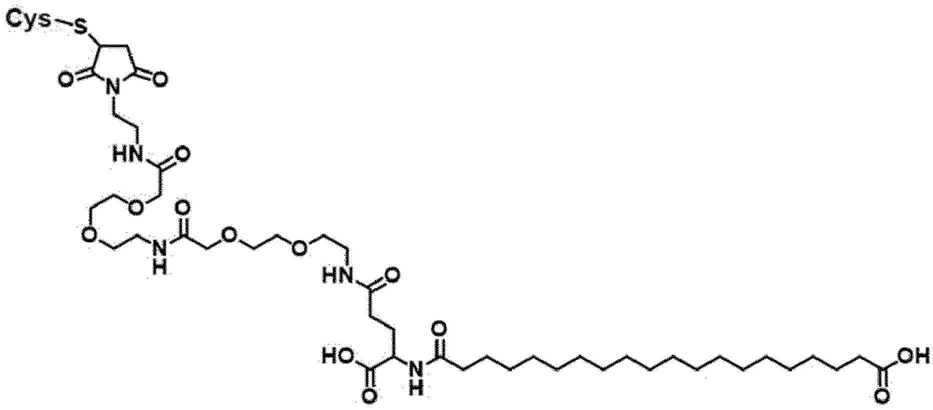

C$^{\#}$ (Cys-B3)

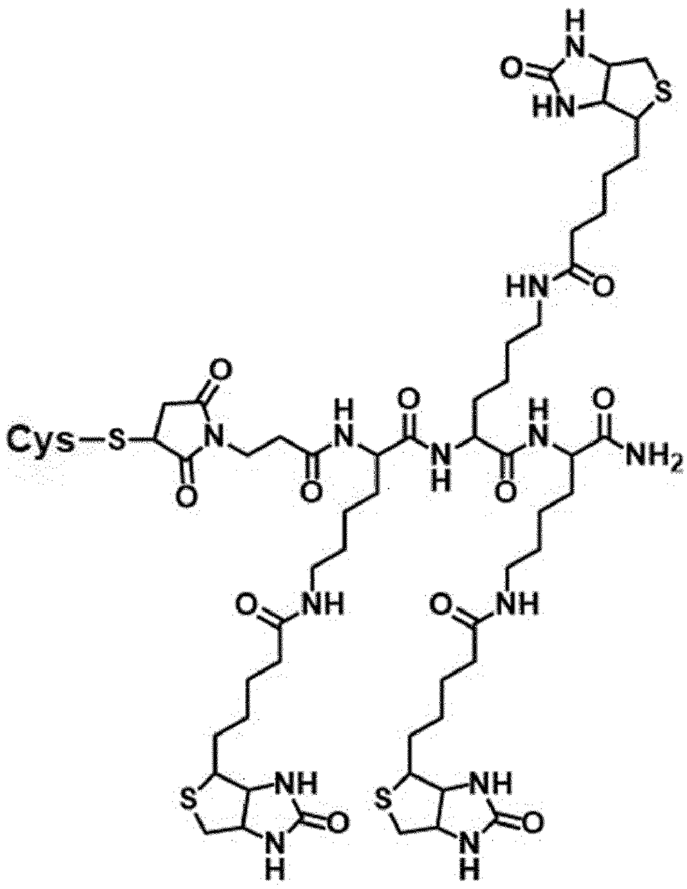

C$^{\#\#}$ (Cys-EG2-Glu-C18-B3)



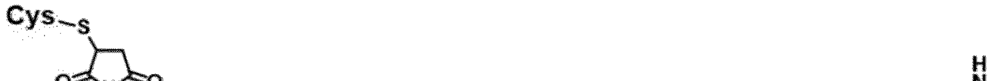

**Excipient**

As excipients, the substances shown in Table 2 below were used. Each excipient is as shown in Table 2.

[Table 2]

| Category | Excipient |
|---|---|
| Excipient A: Bile acid derivative | Chenodeoxycholic acid or its salt (CDC) |
| | Deoxycholic acid or its salt (DC) |
| | Cholic acid or its salt (CA) |
| | Ursodeoxycholic acid or its salt (UDC) |
| | Glycholic acid or its salt (GC) |
| | Taurocholic acid or its salt (TC) |
| Excipient B: | Gallic acid (GA) |
| | Propyl gallate (PG) |
| | Ethylenediaminetetraacetic acid (EDTA) |
| | Camostat mesylate (CM) |
| | Citric acid |
| | Soybean Kunitz trypsin inhibitor (SBTI) |
| | 2-(3,4,5-trihydroxybenzoyl)oxyethyl 3,4,5-trihydrobenzoate |

**<Embodiment 2: Confirmation of tight junction openness of bile acid derivatives and propyl gallate>**

**<Experimental Example 1> Cytotoxicity of bile acid derivatives in Caco-2 cells**

The cytotoxicity of excipients was evaluated using Caco-2 cells using the following method. First, to check the amount of intracellular accumulation, Caco-2 cells were distributed in a 96-well plate at $1 \times 10^5$ per well and cultured in a $CO_2$ incubator at a temperature of 37 °C. The culture solution from each well is removed and replacement by a new culture medium is performed. After that, 2.5 μE of the prepared excipient was added and this was cultured in a $CO_2$ incubator at 37°C. After 2 hours, each well was treated with 100 μL of luciferase reagent (Promega, #G7571) and incubated at room temperature. After 10 minutes, the luminescence value of each well was measured. Cell viability (%) was calculated as follows, and the analysis results are as shown in FIG. 1. As shown in FIG. 1, the cytotoxicity of DC and CDC was similar, and UDC was confirmed to have the least cytotoxicity.

$$\text{Cell viability (\%)} = (\text{luminescence value/control luminescence value}) \times 100$$

**<Experimental Example 2> Confirmation of tight junction openness of bile acid derivatives and propyl gallate in Caco-2 cells.**

Using the monolayer membrane of Caco-2 cells, the tight junction openness and non-tight junction openness of the excipient was evaluated through the change in TEER value. First, to form a Caco-2 cell monolayer, $1.5 \times 10^5$cells per well was dispensed in a 12-Transwell plate and cultured for 3 to 4 weeks under 37°C $CO_2$ conditions. For the first week, the culture medium was changed once every two days, and thereafter, the culture medium was changed every three days. In the experiment, samples between 3 and 4 weeks after seeding were used. To evaluate the degree of damage to the Caco-2 cell monolayer by excipients, the degree of cells cultured as a monolayer on a semi-permeable membrane was measured by the difference in electrical resistance between the apical side and the basolateral side. First, the Transwell to be used in each test is washed using a washing buffer (HBSS), replaced by culture medium, and equilibrated in a 37°C $CO_2$ incubator for 15 minutes. After adding 500 μL of the prepared excipient to the apical side and treating the basolateral side with 1.5 mL of culture medium containing no excipient, changes in TEER values were measured using a Millicell ERS-2 Voltohmmeter at each time point (0, 5, 15, 30, 45, 60, 90, 120 min) from the time of treatment. The TEER change (%) was calculated as follows, and the analysis results are as shown in FIG. 2.

$$\text{TEER change (\%)} = (\text{TEER value/control TEER value}) \times 100$$

As shown in FIG. 2, according to the change in TEER value, the excipients having tight junction openness were CDC and DC, and the excipients having non-tight junction openness were UDC and PG. It was confirmed that even excipients having tight junction openness have reversible tight junction openness that returns to normal levels within 60 to 120 minutes after treatment.

**<Embodiment 3> Measurement of Caco-2 cell membrane permeation pathway of bile acid derivatives**

Using the Caco-2 cell monolayer, changes in the permeability of markers for each permeation pathway due to excipients were evaluated using the following method. First, to form a Caco-2 cell monolayer, $1.5 \times 10^5$ cells per well were dispensed in a 12-Transwell plate and cultured for 3 to 4 weeks under 37°C $CO_2$conditions. For the first week, the culture medium was changed once every two days, and thereafter, the culture medium was changed every three days. In the experiment, samples between 3 and 4 weeks after seeding were used. To verify the formation of a cell monolayer, TEER values and Lucifer yellow values were measured, and only cell monolayer membranes having a TEER value of 300 $\Omega \cdot cm^2$ or more and a measured transmittance of Lucifer yellow within 3% were used in the test. After the Transwells to be used for each test were washed using transport media (HBSS) and incubated in a 37°C $CO_2$ incubator for 1 hour, 500 $\mu$L of bile acid derivatives and permeability marker were added to the apical side, and 1.5 mL of drug-free transport media was treated onto the basolateral side, and incubation was performed for 2 hours in a 37°C $CO_2$ incubator thereafter. At this time, fluorescein isothiocyanate-dextran (FD4) was used as a paracellular marker, and metoprolol was used as a transcellular marker to confirm the change in permeability coefficient according to bile acid derivative treatment. After 2 hours, 1 mL each was sampled from the basolateral side, and the permeability coefficient (Papp value) of the drug was measured using enzyme-linked immunosorbent assay (ELISA) and liquid chromatography-mass spectrometry (HPLC). The permeability coefficient (Papp) value was calculated as follows, and the analysis results are as shown in FIG. 3.

-

$$\text{Papp } (10^{-7}\text{ cm/s}) = (dC_r/d_t) \text{ x } V_r/ (A \text{ x } C_0)$$

(*$dC_r$-concentration of the permeated sample, dt-drug treatment time, $V_r$-basolateral volume, A-Transwell area, $C_0$-initial applied drug concentration)

As shown in FIG. 3, in the Caco-2 test system, while CDC promotes both paracellular transport and transcellular transport. In the case of UDC, it was found that there was no enhancing effect on the two transport mechanisms.

**<Embodiment 4> Preparation of a formulation containing a large physiologically active substance (compounds 1 and 14) and one type of bile acid and measurement of Caco-2 cell membrane permeability of the large physiologically active substance in the formulation**

First, to form a Caco-2 cell monolayer, $1.5 \times 10^5$ cells per well were dispensed in a 12-Transwell plate and this was cultured for 3 to 4 weeks under 37°C $CO_2$ conditions. For the first week, the culture medium was changed once every two days, and thereafter, the culture medium was changed every three days. In the experiment, samples between 3 and 4 weeks after seeding were used. To verify the formation of a cell monolayer, TEER values and Lucifer yellow values were measured; only cell monolayer membranes having a TEER value of 300 $\Omega \cdot cm^2$ or more and a measured transmittance of Lucifer yellow within 3% were used in the test. After washing the Transwell to be used for each test using transport media (HBSS), incubation was performed for 1 hour in a 37°C $CO_2$ incubator; then, 500 $\mu$L each of large physiologically active substance (50 $\mu$M) and bile acid derivatives (100 $\mu$M) were added to the apical side, and 1.5 mL of drug-free transport media was treated onto the basolateral side. Subsequently, incubation was performed for 2 hours in a 37°C $CO_2$ incubator. After 2 hours, 1 mL each was sampled from the basolateral side, and the permeability coefficient (Papp value) of the drug was measured using enzyme-linked immunosorbent assay (ELISA). The permeability coefficient (Papp) value was calculated as follows, and the analysis results are as shown in FIG. 4.

-

$$\text{Papp } (10^{-7}, \text{ cm/s}) = (dC_r/d_t) \text{ x } V_r / (A \text{ x } C_0)$$

(*$dC_r$-concentration of the permeated sample, dt-drug treatment time, $V_r$-basolateral volume, A-Transwell area, $C_0$-initial applied drug concentration)

As shown in FIG. 4, through this experimental example, it was confirmed that in the Caco-2 test, in the case of UDC, the effect of improving the permeability of large physiologically active substances was not observed, while CDC had an effect of improving the permeability of large physiologically active substances.

**<Embodiment 5> Preparation and measurement of intestinal absorption rate of a formulation containing a large physiologically active substance (compound 6), one bile acid derivative, and propyl gallate**

A biotinyl exenatide derivative (Compound 6) was formulated by dissolving in the corresponding vehicle (Saline or 0.5% CMC in saline) with the excipient composition shown in Table 2. and pharmaceutical behavior according to intestinal absorption was compared through intraduodenal administration in Sprague Dawley rats. The results are as shown in Table 3 below.

[Table 3]

| Test substance (mg/kg) | | Excipient (mg/kg) | | | | | Weight ratio (large physiologically active substance : excipient) | BA (%) |
|---|---|---|---|---|---|---|---|---|
| Test substance | Administration dose | Monosaccharide | | PG | Excipient dose | Excipient weight ratio (Monosaccharide: PG) | | |
| | | Type | Administration amount | | | | | |
| Compound 6 | 0.55 | CDC | 17 | 0 | 17 | - | 1:30.9 | 1.0 |
| | 0.55 | CDC | 34 | 0 | 34 | - | 1:61.8 | 1.7 |
| | 0.55 | CDC | 68 | 0 | 68 | - | 1:123.6 | 0.4 |
| | 0.55 | CDC | 17 | 8.5 | 25.5 | 2:1 | 1:46.4 | 3.4 |
| | 0.55 | CDC | 34 | 17 | 51 | 2:1 | 1:92.7 | 7.1 |
| | 0.55 | CDC | 68 | 34 | 102 | 2:1 | 1:185.5 | 7.8 |
| | 0.55 | DC | 16 | 0 | 16 | - | 1:29.1 | 0.1 |
| | 0.55 | DC | 32 | 0 | 32 | - | 1:58.2 | 0.2 |
| | 0.55 | DC | 65 | 0 | 65 | - | 1:118.2 | 0.3 |
| | 0.55 | DC | 16 | 8.5 | 24.5 | 1.9:1 | 1:44.5 | 7.8 |
| | 0.55 | DC | 32 | 17 | 49 | 1.9:1 | 1:89.1 | 13.1 |
| | 0.55 | DC | 65 | 34 | 99 | 1.9:1 | 1:180 | 16.3 |
| | 0.55 | CA | 35 | 0 | 35 | - | 1:63.6 | 0.1 |
| | 0.55 | CA | 71 | 0 | 71 | - | 1:129.1 | 0. 5 |
| | 0.55 | CA | 18 | 8.5 | 26.5 | 2.1:1 | 1:48.2 | 1.0 |
| | 0.55 | CA | 35 | 17 | 52 | 2.1:1 | 1:94.5 | 3.1 |
| | 0.55 | CA | 71 | 34 | 105 | 2.1:1 | 1:190.9 | 12.4 |
| | 0.55 | UDC | 17 | 0 | 17 | - | 1:30.9 | 0.1 |
| | 0.55 | UDC | 34 | 0 | 34 | - | 1:61.8 | 0.2 |
| | 0.55 | UDC | 68 | 0 | 68 | - | 1:123.6 | 0.7 |
| | 0.55 | UDC | 17 | 8.5 | 25.5 | 2:1 | 1:46.4 | 0.3 |
| | 0.55 | UDC | 34 | 17 | 51 | 2:1 | 1:92.7 | 3.9 |

(continued)

| Test substance (mg/kg) | | Excipient (mg/kg) | | | | | Weight ratio (large physiologically active substance : excipient) | BA (%) |
|---|---|---|---|---|---|---|---|---|
| Test substance | Administration dose | Monosaccharide | | PG | Excipient dose | Excipient weight ratio (Monosaccharide: PG) | | |
| | | Type | Administration amount | | | | | |
| | 0.55 | UDC | 68 | 34 | 102 | 2:1 | 1:185.5 | 6.9 |
| | 0.55 | GC | 21 | 0 | 21 | - | 1:38.2 | 0.1 |
| | 0.55 | GC | 42 | 0 | 42 | - | 1:76.4 | 0.1 |
| | 0.55 | GC | 83 | 0 | 83 | - | 1:150.9 | 0.2 |
| | 0.55 | GC | 21 | 8.5 | 29.5 | 2.5:1 | 1:53.6 | 0.1 |
| | 0.55 | GC | 42 | 17 | 59 | 2.5:1 | 1:107.3 | 0.3 |
| | 0.55 | GC | 83 | 34 | 117 | 2.4:1 | 1:212.7 | 4.4 |
| | 0.55 | TC | 22 | 0 | 22 | - | 1:40 | 0.4 |
| | 0.55 | TC | 44 | 0 | 44 | - | 1:80 | 0.1 |
| | 0.55 | TC | 88 | 0 | 88 | - | 1:160 | 0.8 |
| | 0.55 | TC | 44 | 17 | 61 | 2.6:1 | 1:110.9 | 1.5 |
| | 0.55 | TC | 88 | 34 | 122 | 2.6:1 | 1:221.8 | 0.9 |

**<Embodiment 6> Evaluation of the stability of the large physiologically active substance in a formulation containing the large physiologically active substance (Compound 6), one bile acid derivative, and propyl gallate**

For three types of bile acid derivatives with high bioavailability through intestinal absorption, the stability of large physiologically active substances was evaluated in one type of bile acid derivative and propyl gallate formulation. Compound 6 was stored at 40 degrees for 2 weeks with the excipient composition shown in Table 3, and then dissolved in the corresponding solvent (0.02% polysorbate 80 in 10 mM PBS (pH 7.4)). Afterwards, the solution was filtered and the change in purity of the large physiologically active substance (Compound 6) was analyzed by HPLC, and the results are shown in Table 4. As a result of analyzing the change in purity of the test substance after storing it at 40 degrees for 2 weeks, it was confirmed that the stability of the large physiologically active substance (Compound 6) in the DC and propyl gallate compositions was the lowest.

[Table 4]

| Test substance (mg) | Excipient (mg) | | | | Test substance change in purity (%) |
|---|---|---|---|---|---|
| Compound 6 | CDC | DC | UDC | PG | |
| 2.5 | 200 | - | - | 200 | 96.2 |
| 2.5 | - | 200 | - | 200 | 91.7 |
| 2.5 | - | - | 200 | 200 | 97.3 |

**<Embodiment 7> Preparation and measurement of intestinal absorption rate of a formulation containing a large physiologically active substance, one bile acid derivative, and propyl gallate**

Materials containing the compounds in Table 1 were formulated by dissolving them in the corresponding vehicle (0.02% polysorbate 80 in saline) with the excipient composition shown in Table 4. After administration to the duodenum of laboratory rats (SD rats), the pharmaceutical behavior was compared. The results are as shown in Table 5.

[Table 5]

| Test substance (mg/kg) | | Excipient (mg/kg) | | | | Weight ratio (large physiologically active substance : excipient) | $AUC_{last}$ (hr*ng/mL) | BA (%) |
|---|---|---|---|---|---|---|---|---|
| Compound | Administration dose | CDC | PG | Excipient dose | Excipient weight ratio (CDC : PG) | | | |
| 5 | 1.5 | 68 | 34 | 102 | 2:1 | 1:68 | 5960.6 ± 3304.9 | 10.0 ± 5.6* |
| 7 | 1.7 | 68 | 34 | 102 | 2:1 | 1:60 | 196.0 ± 67 | - |
| 9 | 1.9 | 68 | 34 | 102 | 2:1 | 1:53.7 | 12976.4 ± 10198 | 17.4 |
| 10 | 1.7 | 68 | 34 | 102 | 2:1 | 1:60 | 15506.1 ± 4841 | 5.2 |
| 11 | 1.9 | 68 | 34 | 102 | 2:1 | 1:53.7 | 6383.1 ± 2305 | - |
| 12 | 1.9 | 68 | 34 | 102 | 2:1 | 1:53.7 | 1273.4 ± 533 | - |
| 13 | 1.7 | 68 | 34 | 102 | 2:1 | 1:60 | 5011.7 ± 1483.3 | 2.8 ± 0.5 |
| 14 | 1.7 | 68 | 34 | 102 | 2:1 | 1:60 | 6726.6 ± 1216.3 | - |
| (*Relative bioavailability to subcutaneous administration) | | | | | | | | |

**<Embodiment 8> Preparation and measurement of intestinal absorption rate of a formulation containing a large physiologically active substance (Compound 10), one or more bile acid derivatives, and propyl gallate**

A large physiologically active substance (Compound 10) was formulated by dissolving in a vehicle with the excipient composition shown in Table 6. At this time, the vehicle was formulated by appropriately mixing polysorbate 80, propylene glycol, CMC, saline, or phosphate buffer solution. After administering the above formulation to the duodenum of experimental rats, the pharmaceutical behavior was compared. The results are as shown in Table 6 below.

[Table 6]

| Test substance (mg/kg) | | Excipient (mg/kg) | | | | | Weight ratio (large physiologically active substance excipient) | BA (%) |
|---|---|---|---|---|---|---|---|---|
| Test substance | Administration dose | Monosaccharide | | PG | Excipient dose | Excipient weight ratio (Monosaccha ride: PG) | | |
| | | Type | Administration dose | | | | | |
| Compound 10 | 1.7 | CDC | 34 | - | 34 | - | 1:20 | 0.9 |
| | 1.7 | CDC | 68 | - | 68 | - | 1:40 | 0.4 |
| | 1.7 | UDC | 68 | - | 68 | - | 1:40 | 0.8 |
| | 1.7 | UDC | 102 | - | 102 | - | 1:60 | 1.1 |
| | 1.7 | CDC +UDC | 34+68 | - | 102 | - | 1:60 | 2.3 |
| | 1.7 | CDC +UDC | 34+102 | - | 136 | - | 1:80 | 2.0 |
| | 1.7 | - | - | 68 | 68 | - | 1:40 | 0.6 |
| | 1.7 | CDC | 17 | 68 | 85 | 1:4 | 1:50 | 2.4 |
| | 1.7 | CDC | 34 | 68 | 102 | 1:2 | 1:60 | 4.0 |
| | 1.7 | CDC | 68 | 34 | 102 | 2:1 | 1:60 | 5.2 |
| | 1.7 | UDC | 34 | 68 | 102 | 1:2 | 1:60 | 4.1 |
| | 1.7 | DC | 17 | 34 | 51 | 1:2 | 1:30 | 2.9 |
| | 1.7 | DC | 34 | 68 | 102 | 1:2 | 1:60 | 6.4 |
| | 1.7 | DC | 68 | 34 | 102 | 2:1 | 1:60 | 3.9 |
| | 1.7 | CDC +UDC | 34+68 | 34 | 136 | 3:1 | 1:80 | 5.9 |
| | 1.7 | CDC +UDC | 34+68 | 68 | 170 | 1.5:1 | 1:100 | 7.4 |

**<Embodiment 9> Preparation and measurement of intestinal absorption rate of a formulation containing a large physiologically active substance (Compound 24), one bile acid derivative, and propyl gallate**

A large physiologically active substance (Compound 24) was formulated by dissolving it in the corresponding vehicle (0.02% polysorbate 80 in 10 mM PBS (pH 7.4)) with the excipient composition shown in Table 6 above. After administering the above formulation to the duodenum of experimental rats, $C_{max}$ was compared. The corresponding results were compared with the results obtained by administering Compound 23, a large physiologically active substance without a biotin moiety, in the same formulation, and are shown in Table 7 below.

[Table 7]

| Test substance (mg/kg) | | Excipient (mg/kg) | | | | Weight ratio (large physiologically active substance : excipient) | $C_{max}$ (ng/ml) |
|---|---|---|---|---|---|---|---|
| Test substance | Administration dose | CDC | PG | Excipient dose | Excipient weight ratio | | |
| Compound 23 | 5.4 | 68 | 34 | 102 | 2:1 | 1:18.9 | 30.0 |
| Compound 24 | 6.2 | 68 | 34 | 102 | 2:1 | 1:16.4 | 198.1 |

**<Embodiment 10> Preparation and oral absorption rate measurement of a solid formulation containing a large physiologically active substance (Compound 14), one or more bile acid derivatives, and propyl gallate**

A solid formulation of the large physiologically active substance (Compound 14) was prepared with the excipient composition shown in Table 8, and after oral administration to a Beagle dog, the pharmacokinetic behavior was compared.

For the solid formulation, a common excipient (Mannitol, Crosspovidone, Stearate, and the like) used to produce large physiologically active substances, bile acid derivatives, propyl gallate and solid formulations, was mixed therein and this was produced into granules using a dry granulation method and then into tablets using a tablet press. Afterwards, enteric coating was performed using a coating machine. Tablets were produced into immediate-release and sustained-release tablets by adjusting the amount of bonding agent and disintegrating agent. In the immediate-release tablet, more than 80% of the large physiologically active substances were eluted within 60 minutes under elution conditions (pH 6.8, 50 rpm, 37C); in the sustained-release tablet, more than 80% of the large physiologically active substance was eluted within 360 minutes under elution conditions (pH 6.8, 50 rpm, 37C).

[Table 8]

| Category | Test substance (mg/tab) | | Excipient (mg/tab) | | | Weight ratio (large physiologically active substance : excipient) | F (%) |
|---|---|---|---|---|---|---|---|
| | Test substance | Dose | Monosaccharide | | PG | | |
| | | | Type | Dose | | | |
| Sustained release | | 10 | sCDC | 100 | 200 | 1:30 | 0.54 |
| Rapid release | | 10 | sCDC | 100 | 200 | 1:30 | 0.9 |
| Sustained release | | 10 | sCDC | 100 | 200 | 1:50 | 6.2 |
| | | | sUDC | 200 | | | |
| Rapid release | | 10 | sCDC | 100 | 200 | 1:50 | 6.85 |
| | | | sUDC | 200 | | | |
| Sustained release | | 10 | CDC | 100 | 200 | 1:30 | 1.69 |
| Rapid release | | 10 | UDC | 300 | 200 | 1:50 | 1.55 |

(continued)

| Category | Test substance (mg/tab) | | Excipient (mg/tab) | | | Weight ratio (large physiologically active substance : excipient) | F (%) |
|---|---|---|---|---|---|---|---|
| | Test substance | Dose | Monosaccharide | | PG | | |
| | | | Type | Dose | | | |
| Sustained release | Compound 14 | 10 | CDC | 100 | 200 | 1:50 | 2.54 |
| | | | UDC | 200 | | | |
| Rapid release | | 10 | CDC | 100 | 200 | 1:50 | 3.99 |
| | | | UDC | 200 | | | |
| Rapid release | | 10 | CDC | 33 | 200 | 1:29.9 | 1.13 |
| | | | UDC | 66 | | | |
| Rapid release | | 10 | CDC | 66 | 200 | 1:39.9 | 6.76 |
| | | | UDC | 133 | | | |
| Rapid release | | 10 | CDC | 150 | 200 | 1:38 | 5.03 |
| | | | UDC | 30 | | | |
| Rapid release | | 10 | CDC | 200 | 200 | 1:50 | 11.03 |
| | | | UDC | 100 | | | |
| Rapid release | | 10 | CDC | 100 | 100 | 1:40 | 5.99 |
| | | | UDC | 200 | | | |
| Rapid release | | 10 | CDC | 100 | 50 | 1:35 | 0.23 |
| | | | UDC | 200 | | | |
| Rapid release | | 10 | CDC | 100 | 2 | 1:30.2 | 0.56 |
| | | | UDC | 200 | | | |
| Rapid release | | 10 | CDC | 0 | 200 | 1:50 | 1.55 |
| | | | UDC | 300 | | | |
| (PG: Propylgallate, sCDC: Sodium Chenodeoxycholate, sUDC: Sodium ursodeoxycholate) | | | | | | | |

**<Embodiment 11> Measurement of blood sugar regulation ability of a formulation containing a large physiologically active substance (Compound 1), one or more bile acid derivatives, and propyl gallate**

To confirm glucose tolerance, a formulation containing a combination of large physiologically active substance and excipient was orally administered to mice, and then the blood sugar regulation efficacy was measured through an Intraperitoneal Glucose tolerance test (IPGTT). A large physiologically active substance (Compound 1) was formulated by dissolving it in a vehicle (0.02% polysorbate 80 in 10 mM PBS (pH 7.4)) with the excipient composition shown in Table 9 below.

[Table 9]

| | Vehicle | Excipient composition |
|---|---|---|
| Control group | Compound 1, 25 nmol/kg | - |
| Test group | Compound 1,25 nmol/kg | 17 mg/kg CDC, and 34 mg/kg PG |
| Test group | Compound 1, 25 nmol/kg | 17 mg/kg CDC, 34 mg/kg TC, & 34 mg/kg PG |
| Test group | Compound 1, 25 nmol/kg | 17 mg/kg CDC, 34 mg/kg GC, & 34 mg/kg PG |

(continued)

| | Vehicle | Excipient composition |
|---|---|---|
| Test group | Compound 1, 25 nmol/kg | 17 mg/kg CDC, 34 mg/kg DC, & 34 mg/kg PG |
| Test group | Compound 1, 25 nmol/kg | 17 mg/kg CDC, 34 mg/kg UDC, & 34 mg/kg PG |

To measure abdominal glucose tolerance in an animal model, 100 μl of sample (25 nmol/kg, based on Compound 1) was orally administered at -20 minutes to 9-week-old male mice (C57BL/6); then 200 μl of glucose (2 g/kg) was injected intraperitoneally, and changes in blood sugar in blood collected from the tail vein were observed at -20, 0, 20, 40, 60, 90, and 120 minutes. The measurement results are shown in FIG. 5.

As shown in FIG. 5, it was confirmed that the ability to regulate glucose according to oral absorption increased when two types of bile acids rather than one type were used as absorption accelerators. Among the two types of bile acids, it was confirmed that the CDC+DC+propyl gallate and CDC+UDC+propyl gallate formulations had the best blood sugar regulation efficacy.

In addition, the CDC+DC+PG formulation and CDC+UDC+PG formulation, which had the best glucose regulation ability, were prepared and left at room temperature for 1 day, then orally administered to mice, and the blood sugar regulation efficacy was measured through an intraperitoneal glucose tolerance test. Through this, the effect of the stability of the large physiologically active substance in the formulation applicable to the efficacy of the large physiologically active substance was confirmed. As shown in FIG. 6, the results confirmed that after 1 day, the efficacy of the CDC+UDC+PG formulation was maintained, while the efficacy of the CDC+DC+PG formulation decreased to 82%.

### <Embodiment 12> Confirmation of weight loss and appetite suppression effects through oral administration of a large physiologically active substance (Compound 14), one or more bile acid derivatives, and propyl gallate formulation

After oral administration of a formulation containing a large physiologically active substance (Compound 14) and excipients to mice, changes in mouse body weight were confirmed. Specifically, the administered dose of Compound 14 was 1000 nmol/kg, and the excipient concentrations in the administered formulation were CDC 6.8 mg/mL, UDC 13.6 mg/mL, and PG 13.6 mg/mL. The vehicle used 0.02% polysorbate 80 in 10 mM PBS (pH 7.4).

Obese mice were induced by feeding high-fat diet to 6-week-old male mice (C57BL/6) for 16 weeks; then a substance combining biotin and fatty acid moieties (Compound 14) was administered orally daily for 4 weeks using a formulation containing two types of bile acid derivatives and propyl gallate. As a result of checking the weight change, a decrease in feed intake and weight loss were confirmed. The measurement results are as shown in FIGS. 7 and 8.

### <Embodiment 13> Preparation of a formulation containing a large physiologically active substance (Compound 15), one or more bile acid derivatives, and propyl gallate or an enzyme inhibitor and measurement of intestinal absorption rate in rats

A large physiologically active substance (Compound 15) was formulated by dissolving it in the corresponding vehicle (0.02% polysorbate 80 in 10 mM PBS (pH 7.4)) with the excipient composition shown in Table 9. After administering the sample to the duodenum in amounts of 100 and 500 μg/kg, respectively, to laboratory rats (SD rats) weighing about 200 g, serum was collected, and changes in blood drug concentration over time were measured using enzyme-linked immunosorbent assay (ELISA). Blood samples were taken from the jugular vein. The results were calculated as average values, and the results are shown in Table 10.

[Table 10]

| Physiologically active substance | Excipient (mg/kg) | | | | | | | Bioavailability BA (%) |
|---|---|---|---|---|---|---|---|---|
| | Monosaccharide | | PG | EDTA | Camostat mesylate | Citric acid | SBTI | |
| | CDC | UDC | | | | | | |
| Compound 15 | 68 | - | 34 | | | | | 1.07±0.68 |
| | - | 68 | 34 | | | | | 0.96±0.40 |
| | 68 | 68 | 34 | | | | | 1.39±0.27 |
| | 68 | 68 | 68 | - | - | - | - | 1.67±0.29 |
| | 68 | 68 | 68 | 66 | - | - | - | 6.06±2.40 |
| | 68 | 68 | 68 | - | 12 | - | - | 9.30±3.62 |
| | 68 | 68 | 68 | - | - | 220 | - | 0.27±0.16 |
| | 68 | 68 | 68 | - | - | - | 12 | 4.36±1.15 |
| | 68 | 68 | - | - | 12 | - | - | 2.16±1.77 |
| | 68 | 68 | - | - | - | - | 12 | 1.12±0.42 |
| | 68 | 68 | 68 | - | 12 | - | - | 0.25±0.08 |

**<Embodiment 14> Measurement of blood sugar regulation ability according to oral absorption of a formulation containing a large physiologically active substance (Compound 15), one or more bile acid derivatives, and propyl gallate or enzyme inhibitors**

A large physiologically active substance (Compound 15) was formulated by dissolving it in the corresponding vehicle (0.02% polysorbate 80 in 10 mM PBS (pH 7.4)) with the excipient composition shown in Table 10. To confirm glucose tolerance, a formulation containing a combination of large physiologically active substance and excipient was orally administered to mice, and then the blood sugar regulation efficacy was measured through an Intraperitoneal Glucose tolerance test (IPGTT). First, to measure abdominal glucose tolerance in an animal model, 100 $\mu\ell$ of sample (25 nmol/kg, based on Compound 1) was orally administered at -30 minutes to 9-week-old male mice (C57BL/6); then 200μl of glucose (2 g/kg) was injected intraperitoneally, and changes in blood sugar levels were observed in blood collected from the tail vein at -20, 0, 20, 40, 60, 90, and 120 minutes. The measurement results are as shown in Table 11.

[Table 11]

| | Administration route | Dose (mg/kg) | Excipient (mg/kg) | | | | | $AUC_{0\text{-}120min}$ (min*mg/ dL) |
|---|---|---|---|---|---|---|---|---|
| | | | Monosaccharide | | PG | Camostat mesylate | SBTI | |
| | | | CDC | UDC | | | | |
| Control group | Oral | - | - | - | - | - | | 33,935.0± 2,699.1 |
| Compound 15 | Subcut aneous | 0.13 | - | - | - | - | - | 10,669.0± 2,213.0 |
| | Oral | 0.44 | 68 | 68 | 68 | 12 | - | 18,727.0± 4,528.7 |
| | Oral | 1.32 | 68 | 68 | 68 | 12 | - | 14,034.0± 908.3 |
| | Oral | 0.44 | 68 | 68 | 68 | - | 12 | 18,893.0± 3,413.2 |
| | Oral | 1.32 | 68 | 68 | 68 | - | 12 | 15,263.0± 3,010.1 |

**<Embodiment 15> Confirmation of weight loss and appetite suppression effects through oral administration of a large physiologically active substance (Compound 15), one or more bile acid derivatives, and a propyl gallate formulation**

After oral administration of a formulation containing a large physiologically active substance (Compound 15) and excipients to mice, changes in mouse body weight were confirmed. Specifically, the administered dose of Compound 15 was 1000 nmol/kg, and the excipient concentrations in the administered formulation used CDC 6.8 mg/mL, UDC 13.6 mg/mL, and PG 13.6 mg/mL. The vehicle used 0.02% polysorbate 80 in 10 mM PBS (pH 7.4). Obese mice were induced by feeding high-fat diet to 6-week-old male mice (C57BL/6) for 16 weeks; then, a GLP-1 receptor agonist (Compound 15) combined with a fatty acid moiety was orally administered daily for 3 weeks using a bile acid absorption accelerator formulation, and body weight changes were confirmed. As a result, a decrease in feed intake and weight loss were confirmed. The measurement results are as shown in FIGS. 9 and 10.

**<Embodiment 16> Confirmation of weight loss and appetite suppression effects through oral administration of a large physiologically active substance (Compound** 17), **one or more bile acid derivatives, and propyl gallate formulation**

After oral administration of a formulation containing a large physiologically active substance (Compound 17) and excipients to mice, changes in mouse body weight and anti-diabetic efficacy were confirmed. Specifically, the administered dose of Compound 17 used 300 to 1000 nmol/kg, and the excipient concentrations in the administered formulation used CDC 6.8 mg/mL, UDC 13.6 mg/mL, and PG 13.6 mg/mL. The vehicle used 0.02% polysorbate 80 in 10 mM PBS (pH 7.4). Obese mice were induced by feeding high-fat diet to 6-week-old male mice (C57BL/6) for 16 weeks; then weight changes were confirmed by oral administration of the GLP-1/Glucagon receptor dual agonist (Compound 17), which combines biotin and fatty acid moieties, every day for 3 weeks using a bile acid absorption accelerator formulation. A reduction of feed intake, weight loss, and anti-diabetic effect were confirmed. The measurement results are as shown in FIGS. 11 to 13.

**<Embodiment 17> Measurement of blood sugar regulation ability through oral administration of a large physiologically active substance (Compound 18), one or more bile acid derivatives, and propyl gallate formulation**

To check glucose tolerance, after oral administration to mice of a formulation made by combining a GLP-1/Glucagon/GIP receptor triple agonist (Compound 18), which is a large physiologically active substance combined with biotin and a fatty acid moiety, and excipients in Table 12 below, blood sugar regulation efficacy was measured using an intraperitoneal glucose tolerance test (IPGTT).

[Table 12]

| | Vehicle | Excipient Composition |
|---|---|---|
| Control group | Compound 18, 100 nmol/kg | - |
| Test group | Compound 18, 100 nmol/kg | 51 mg/kg CDC, & 34 mg/kg PG |
| Test group | Compound 18, 100 nmol/kg | 17 mg/kg CDC, 34 mg/kg TC, & 34 mg/kg PG |
| Test group | Compound 18, 100 nmol/kg | 17 mg/kg CDC, 34 mg/kg GC, & 34 mg/kg PG |
| Test group | Compound 18, 100 nmol/kg | 17 mg/kg CDC, 34 mg/kg DC, & 34 mg/kg PG |
| Test group | Compound 18, 100 nmol/kg | 17 mg/kg CDC, 34 mg/kg UDC, & 34 mg/kg PG |

First, to measure abdominal glucose tolerance in an animal model, 100 μl of sample (25 nmol/kg, based on Compound 1) was orally administered at -20 minutes to 9-week-old male mice (C57BL/6); then 200 μl of glucose (2 g/kg) was injected intraperitoneally, and changes in blood sugar levels were observed in blood collected from the tail vein at -20, 0, 20, 40, 60, 90, and 120 minutes. The measurement results are as shown in FIG. 14. It was confirmed that the ability to regulate glucose according to oral absorption increased when two types of bile acids were used rather than one type as an absorption enhancer.

**<Embodiment 18> Measurement of blood sugar regulation ability through oral administration of a large physiologically active substance (Compound 19), one or more bile acid derivatives, and propyl gallate formulation**

To check glucose tolerance, after oral administration to mice of a formulation made by combining insulin (Compound 19), which is a large physiologically active substance with a fatty acid moiety, and excipients in Table 13 below, blood sugar regulation efficacy was measured using an intraperitoneal glucose tolerance test (IPGTT).

[Table 13]

| | Vehicle | Excipient Composition |
|---|---|---|
| Control group | Compound 19, 50 nmol/kg | - |
| Test group | Compound 19, 50 nmol/kg | 51 mg/kg CDC, & 34 mg/kg PG |
| Test group | Compound 19, 50 nmol/kg | 17 mg/kg CDC, 34 mg/kg TC, & 34 mg/kg PG |
| Test group | Compound 19, 50 nmol/kg | 17 mg/kg CDC, 34 mg/kg GC, & 34 mg/kg P G |
| Test group | Compound 19, 50 nmol/kg | 17 mg/kg CDC, 34 mg/kg DC, & 34 mg/kg PG |
| Test group | Compound 19, 50 nmol/kg | 17 mg/kg CDC, 34 mg/kg UDC, and 34 mg/kg PG |

First, to measure abdominal glucose tolerance in an animal model, 100 μl of sample (25 nmol/kg, based on Compound 1) was orally administered at -20 minutes to 9-week-old male mice (C57BL/6); then, 200 μl of glucose (2 g/kg) was injected intraperitoneally, and changes in blood sugar in blood collected from the tail vein were observed at -20, 0, 20, 40, 60, 90, and 120 minutes. The measurement results are shown in FIG. 15. It was confirmed that the ability to regulate glucose according to oral absorption increased when two types of bile acids were used rather than one type as an absorption enhancer. Among the two types of bile acids, it was confirmed that the CDC+DC, CDC+UDC, and propyl gallate formulations had the best blood sugar control efficacy.

In addition, the CDC+DC+PG formulation and CDC+UDC+PG formulation, which had the best glucose regulation ability, were prepared and left at room temperature for 1 day, then orally administered to mice, and the blood sugar regulation efficacy was measured through an intraperitoneal glucose tolerance test. Through this, the effect of the stability of the large physiologically active substance in the formulation applicable to the efficacy of the large physiologically active substance was confirmed. As shown in FIG. 16, it was confirmed that, while the efficacy of the CDC+UDC+PG formulation was maintained after 1 day, the CDC+DC+PG formulation decreased the blood sugar reduction effect from 64% to 76% compared to the control group at 20 minutes after administration of the test substance.

**<Embodiment 19> Measurement of blood sugar regulation ability through oral administration of large physiologically active substances (Compounds 20, 21, 22), one or more bile acid derivatives, and propyl gallate formulation**

To check glucose tolerance, after oral administration to mice, a formulation made by combining insulin (Compound 21) with biotin as a large physiologically active substance and insulin (Compound 22) with biotin or fatty acid moieties and excipients was administered to mice, and blood sugar regulation efficacy was measured using an intraperitoneal glucose tolerance test (IPGTT). First, to measure abdominal glucose tolerance in an animal model, 100 μl of sample (25 nmol/kg, based on Compound 1) was orally administered at -20 minutes to 9-week-old male mice (C57BL/6); then 200 μl of glucose (2 g/kg) was injected intraperitoneally, and changes in blood sugar level in blood collected from the tail vein were observed at -20, 0, 20, 40, 60, 90, and 120 minutes. The measurement results are as shown in FIG. 17. It was confirmed that the glucose regulation ability according to oral absorption increased in the case of insulin combined with biotin and fatty acid moieties compared to the case in which only biotin was combined with insulin.

**<Embodiment 20> Measurement of blood sugar regulation ability through oral administration of a large physiologically active substance (Compounds 25 to 37), one or more bile acid derivatives, and propyl gallate formulations**

After a single oral administration of a formulation using a combination of a large physiologically active substance and excipients to mice, body weight changes were confirmed for 24 hours. Amylin derivatives with biotin (compounds 26, 29, 32, and 35) and amylin derivatives with biotin and fatty acid moieties (compounds 27, 30, 33, and 36) were administered orally as a single dose using a bile acid absorption accelerator formulation to determine body weight

changes, and weight loss was confirmed after 24 hours, and the measurement results are shown in Table 14.

[Table 14]

| Compound | Weight loss compared to control group (%) |
|---|---|
| Control group | - |
| 25 | - 2.6±1.1 |
| 26 | - 4.1±13 |
| 27 | - 3.0±4.2 |
| 28 | - 3.1±1.1 |
| 29 | - 4.5±0.9 |
| 30 | - 3.3±1.3 |
| 31 | - 1.1±0.8 |
| 32 | - 2.0±0.9 |
| 33 | - 2.4±0.8 |
| 34 | - 1.2±0.8 |
| 35 | - 2.6±0.6 |
| 36 | - 3.4±1.3 |

As shown in Examples 1 to 20 above, it was confirmed that, when an excipient is used together compared to when a large physiologically active substance is administered alone, there was an overall improvement in terms of efficacy and pharmacokinetic parameters due to the presence of a large physiologically active substance.

Those skilled in the art will recognize, or be able to confirm using no more than routine experimentation, many equivalents to the specific examples of the invention described herein. Such equivalents are intended to be encompassed by the following claims. The description of the present application described above is for illustrative purposes, and those skilled in the art will be able to understand that the present application can be easily modified into other specific forms without changing its technical idea or essential features. Therefore, the examples described above should only be understood in all respects as illustrative and not restrictive. For example, each component described as a monotype may be implemented by being dispersed, and similarly, components described as being dispersed may also be implemented in a combined form. The meaning and scope of the patent claims described below, and all changes or modified forms derived from the equivalent concept thereof, may be interpreted as being included in the scope of the present invention.

[Industrial Applicability]

The present invention is able to increase the absorption rate of large physiologically active substances in the body, and can be available for use in the pharmaceutical industry.

## Claims

**1.** Pharmaceutical composition, comprising

(i) a large physiologically active substance; and (ii) excipient A comprising a bile acid derivative; or
(iii) excipient B comprising a compound or a derivative thereof having CYP450 inhibition, antioxidant effect, or gastrointestinal enzyme activity inhibition effect.

**2.** The pharmaceutical composition according to Claim 1,
wherein the (i) large physiologically active substance is a polypeptide, protein, polysaccharide, nucleotide, or analog thereof.

**3.** The pharmaceutical composition according to Claim 1,

wherein, in excipient A, the bile acid derivative is at least one selected from the group consisting of glycocholic acid, glycocholicchenodeoxycholic acid, taurocholic acid, deoxycholic acid, taurodeoxycholic acid, cholic acid, chenodeoxycholic acid, ursodeoxycholic acid, lithocholic acid, Dehydrocholic acid and pharmaceutically acceptable salts thereof.

**4.** The pharmaceutical composition according to Claim 1,
wherein, in excipient A, the bile acid derivative is at least one selected from the group consisting of glycocholic acid, taurocholic acid, deoxycholic acid, cholic acid, chenodeoxycholic acid, ursodeoxycholic acid and pharmaceutically acceptable salts thereof.

**5.** The pharmaceutical composition according to Claim 1,
wherein, in excipient A, the bile acid derivative is at least one selected from the group consisting of chenodeoxycholic acid, ursodeoxycholic acid, and pharmaceutically acceptable salts thereof.

**6.** The pharmaceutical composition according to Claim 1,
wherein, in excipient A, the bile acid derivative comprises a tight junction open bile acid derivative.

**7.** The pharmaceutical composition according to Claim 1,
wherein, in excipient A, the bile acid derivative comprises a non-tight junction open bile acid derivative.

**8.** The pharmaceutical composition according to Claim 1,
wherein, in excipient A, the bile acid derivative comprises a tight junction open bile acid derivative A-1, and a non-tight junction open bile acid derivative A-2.

**9.** The pharmaceutical composition according to Claim 1,
wherein the excipient B comprises a compound or a derivative thereof having at least one of CYP450 inhibition, antioxidant effect, or gastrointestinal enzyme activity inhibition effect.

**10.** The pharmaceutical composition according to Claim 1,
wherein, in the excipient B, the (iii) compound or derivative thereof having CYP450 inhibition, antioxidant effect, or gastrointestinal enzyme activity inhibition effect is at least one selected from the group consisting of CYP450 inhibitory compounds, antioxidant compounds, proteolytic enzyme inhibitory compounds, and pharmaceutically acceptable salts thereof.

**11.** The pharmaceutical composition according to Claim 10,
wherein the CYP450 inhibitory compound is propyl gallate or a pharmaceutically acceptable salt thereof.

**12.** The pharmaceutical composition according to Claim 10,
wherein the antioxidant compound is at least one selected from the group consisting of gallic acid, caffeic acid, lipoic acid, citric acid, acetyl carnitine, acetyl cysteine, glutathione, ascorbyl compounds, tocopheryl compounds, and pharmaceutically acceptable salts thereof.

**13.** The pharmaceutical composition according to Claim 10,
wherein the proteolytic enzyme inhibitory compound is at least one selected from the group consisting of propyl gallate, Aprotinin, Camostat mesylate, Gabexate mesylate, Soybean Kunitz trypsin inhibitor (SBTI), Soybean Kunitz trypsin-chymotrypsin inhibitor (SBTCI), Soybean Bowman-Birk inhibitor, EDTA, Bacitracin, ovomucoid, citric acid, and pharmaceutically acceptable salts thereof.

**14.** The pharmaceutical composition according to Claim 1,
wherein the excipient B is propyl gallate; and at least one selected from the group consisting of Camostat mesylate, citric acid, soy trypsin inhibitor or EDTA.

**15.** The pharmaceutical composition according to Claim 1,
wherein the excipient B is at least one selected from the group consisting of propyl gallate, Camostat mesylate, or a pharmaceutically acceptable salt thereof.

**16.** The pharmaceutical composition according to Claim 1,

wherein in the excipient A, the bile acid derivative is at least one selected from the group consisting of chenodeoxycholate, ursodeoxycholate and pharmaceutically acceptable salts thereof; and
the excipient B is at least one selected from the group consisting of propyl gallate, Camostat mesylate, and pharmaceutically acceptable salts thereof.

**17.** The pharmaceutical composition according to Claim 1,
wherein the weight ratio of the (i) large physiologically active substance and (ii) excipient A is 1:1 to 1500.

**18.** The pharmaceutical composition according to Claim 1,
wherein the (ii) excipient A comprises two or more bile acid derivatives, and the weight ratio of each is 1 to 1500 compared to the weight of the large physiologically active substance.

**19.** The pharmaceutical composition according to Claim 1,
wherein the weight ratio of the (i) large physiologically active substance and the (iii) excipient B is 1:0.1 to 300.

**20.** The pharmaceutical composition of any one of claims 1 to 19,
wherein the pharmaceutical composition is administered orally.

**21.** A method of producing pharmaceutical composition, comprising a step of mixing (i) a large physiologically active substance; and (ii) excipient A comprising a bile acid derivative, or (iii) excipient B comprising a compound or a derivative thereof having CYP450 inhibition, antioxidant effect, or gastrointestinal enzyme activity inhibition effect.

[FIG. 1]

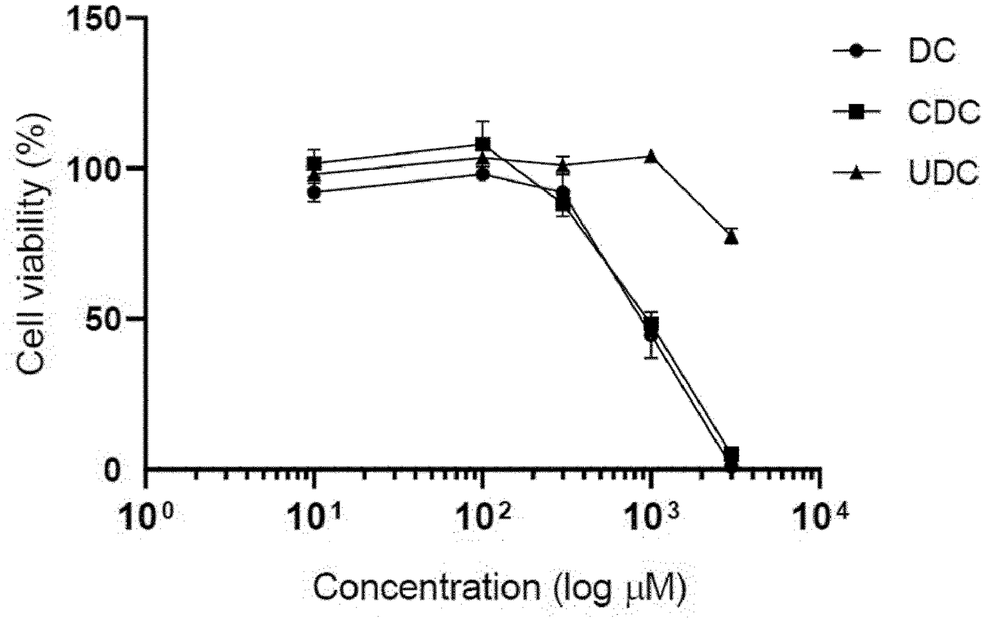


[FIG. 2]

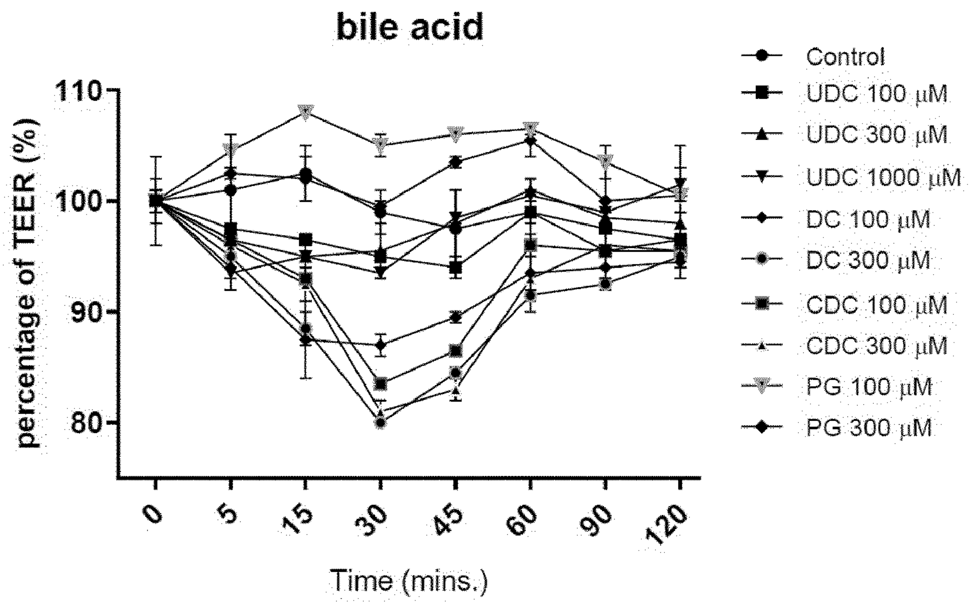


[FIG. 3]

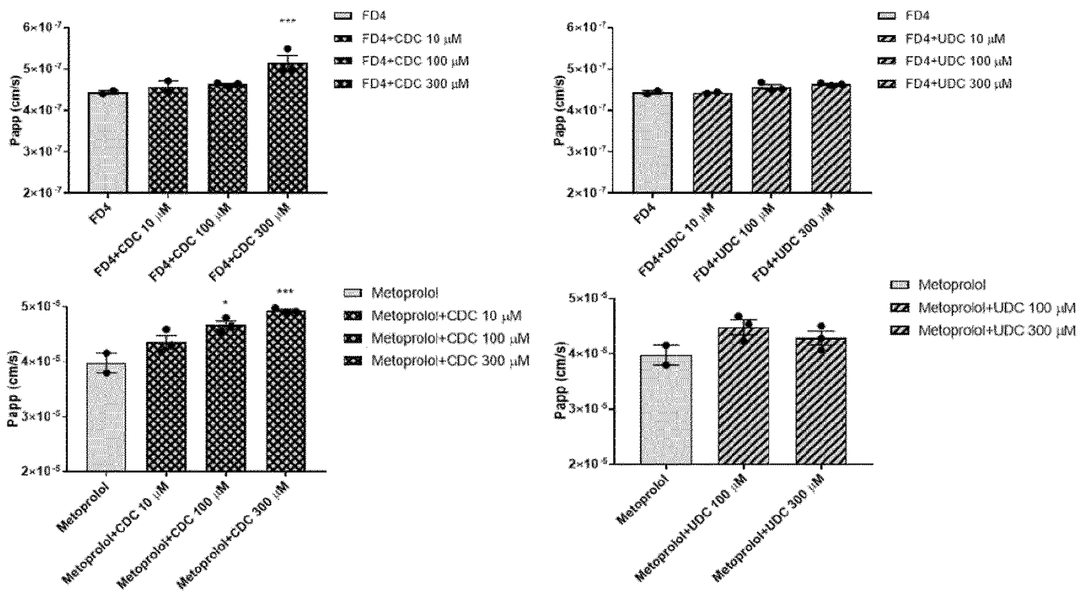


[FIG. 4]

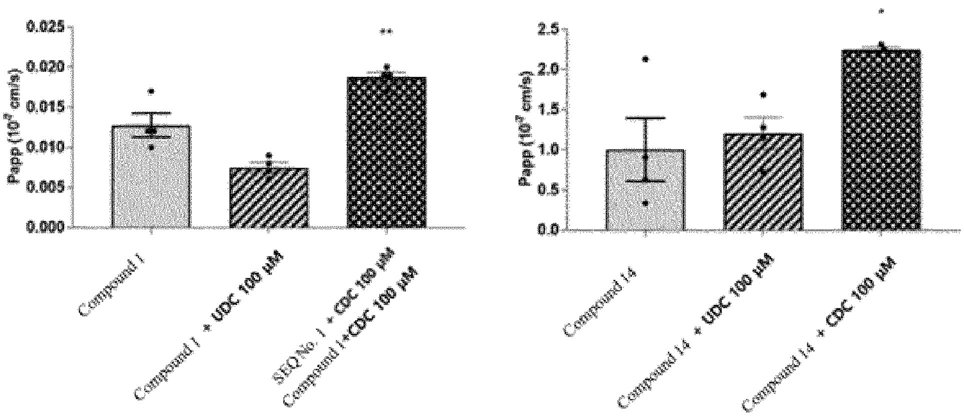


[FIG. 5]

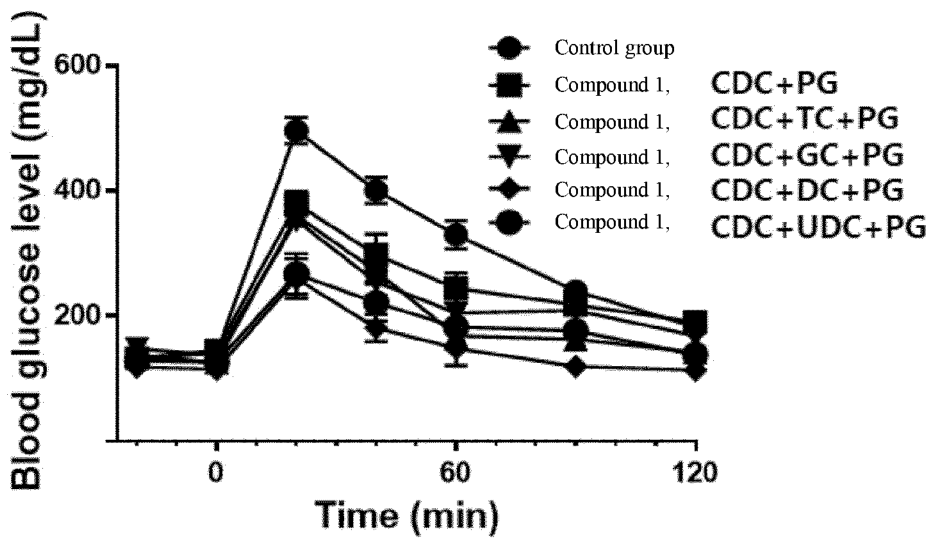


[FIG. 6]

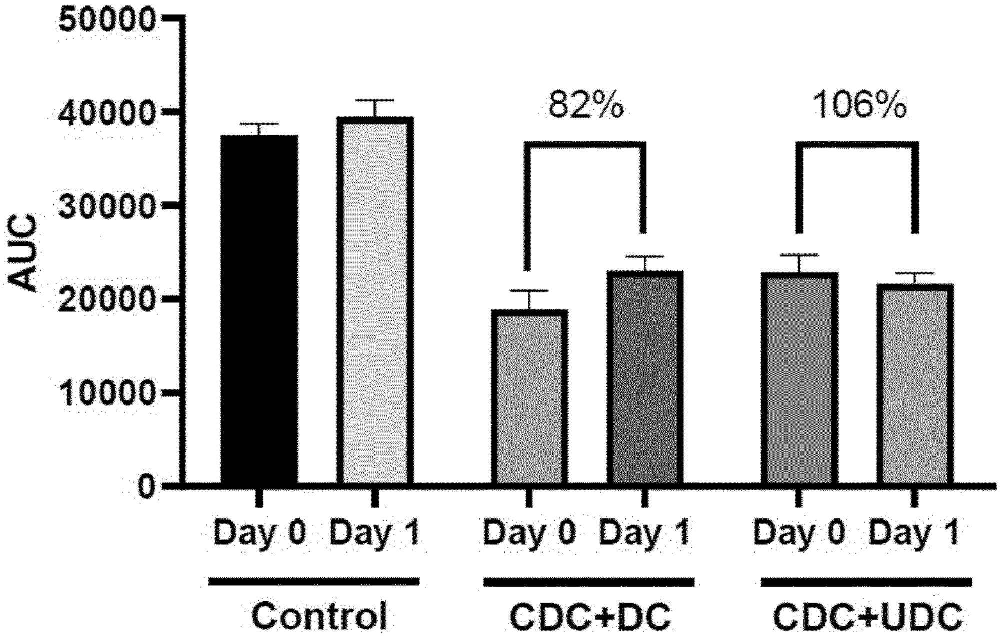
50000
40000
30000
20000
10000
0
AUC
82%
106%
Day 0
Day 1
Day 0
Day 1
Day 0
Day 1
Control
CDC+DC
CDC+UDC

[FIG. 7]

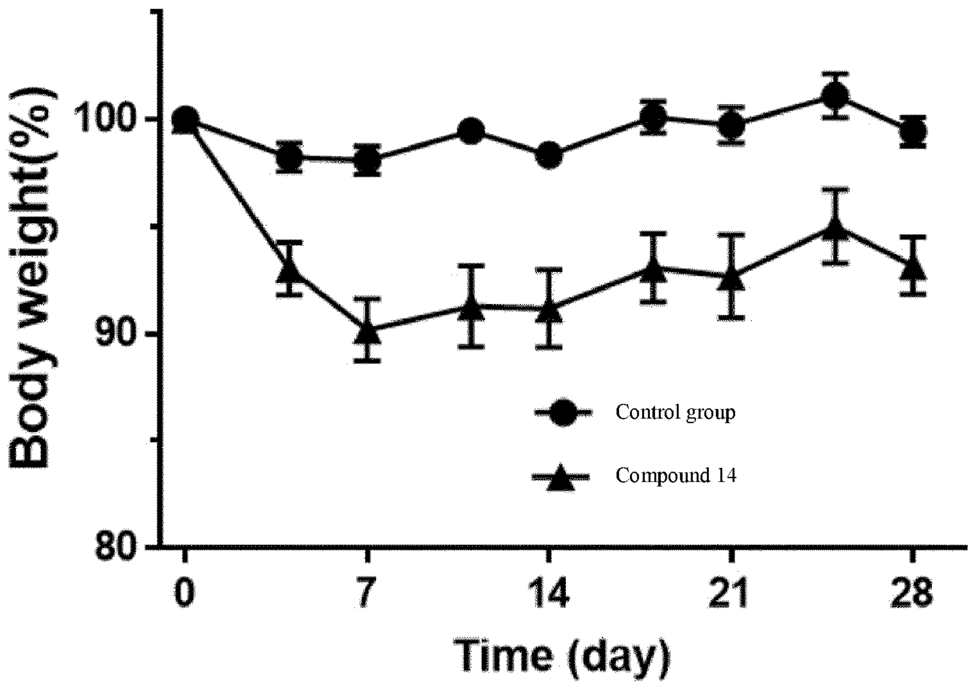
Body weight(%)
100
90
80
0
7
14
21
28
Time (day)
Control group
Compound 14

[FIG. 8]

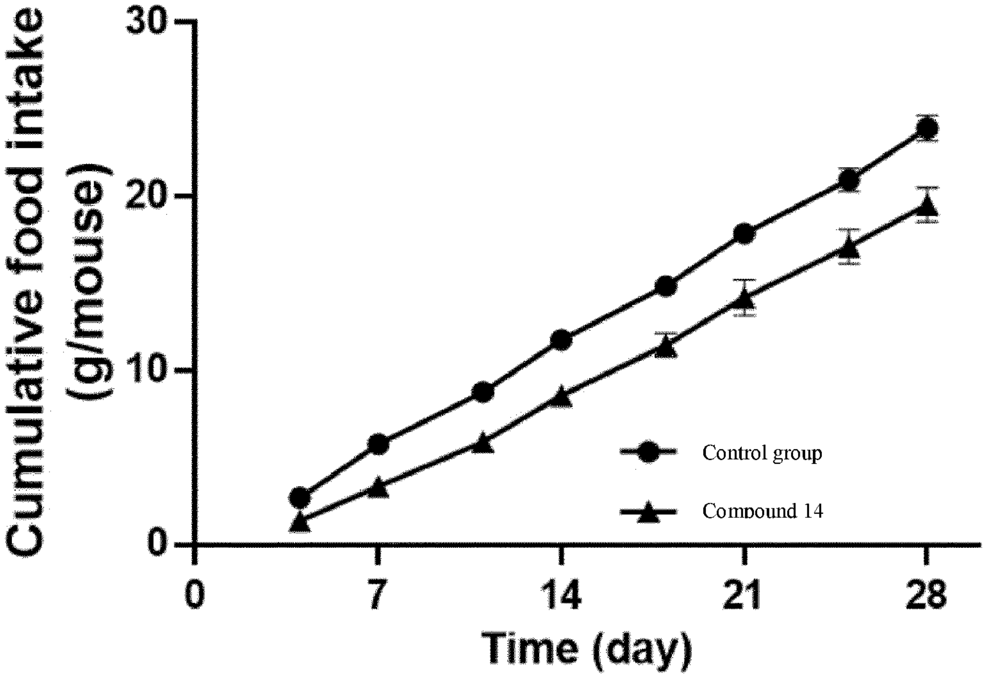
Cumulative food intake (g/mouse)
30
20
10
0
0
7
14
21
28
Time (day)
Control group
Compound 14

[FIG. 9]

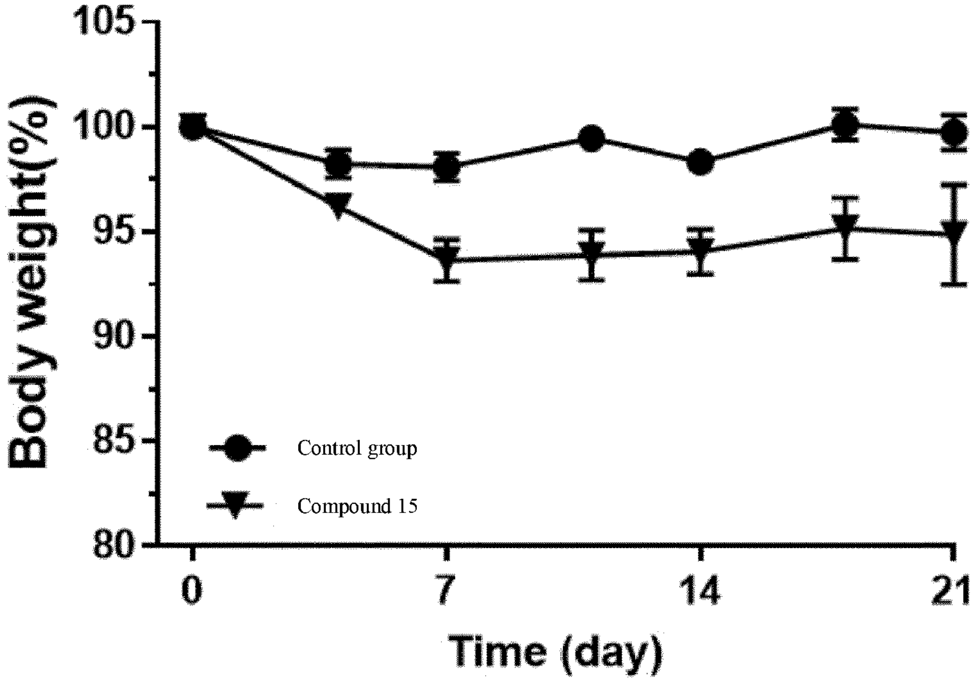
Body weight(%)
105
100
95
90
85
80
0
7
14
21
Time (day)
Control group
Compound 15

[FIG. 10]

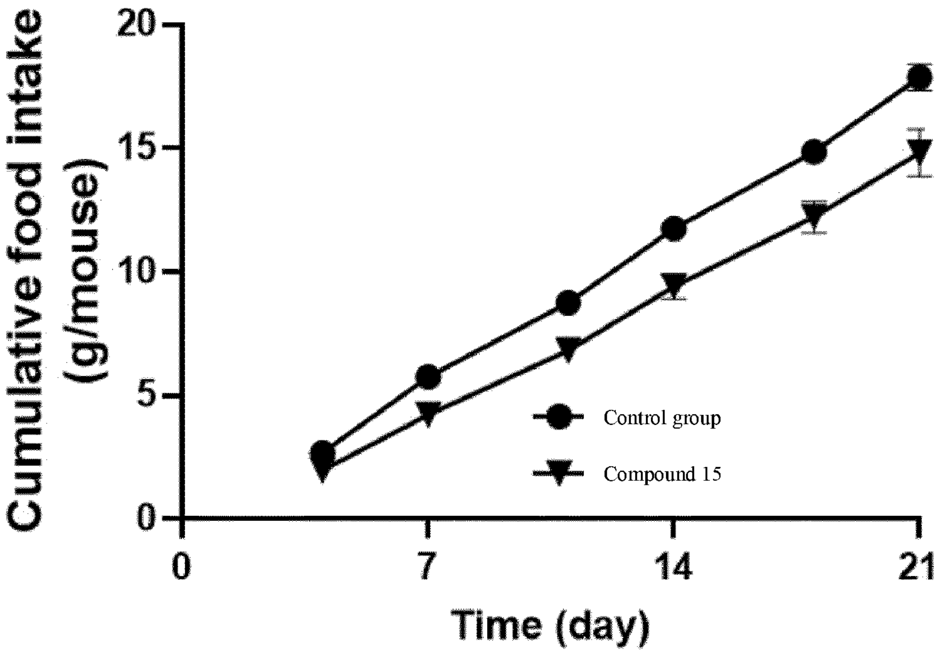


[FIG. 11]

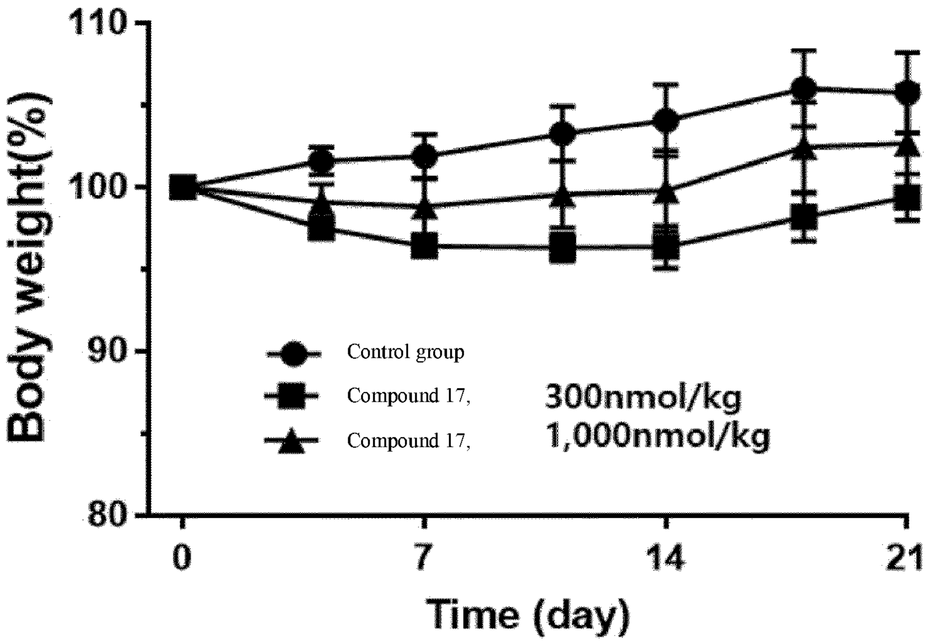


[FIG. 12]

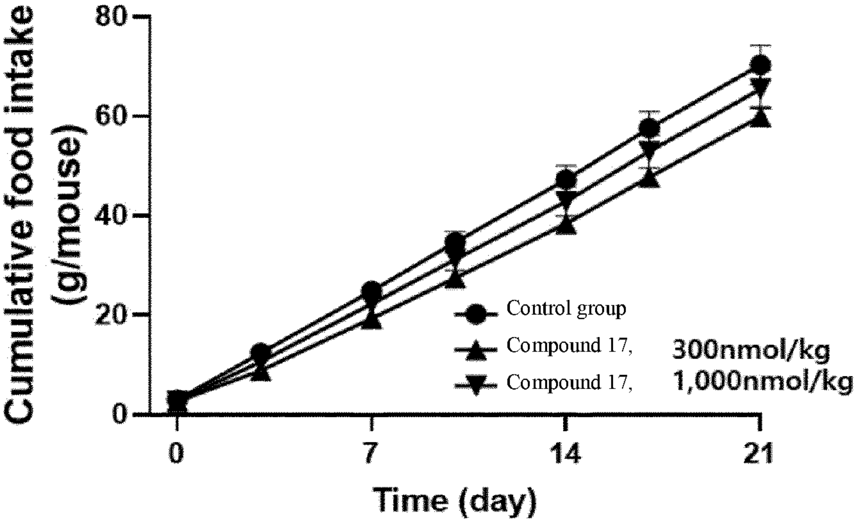


[FIG. 13]

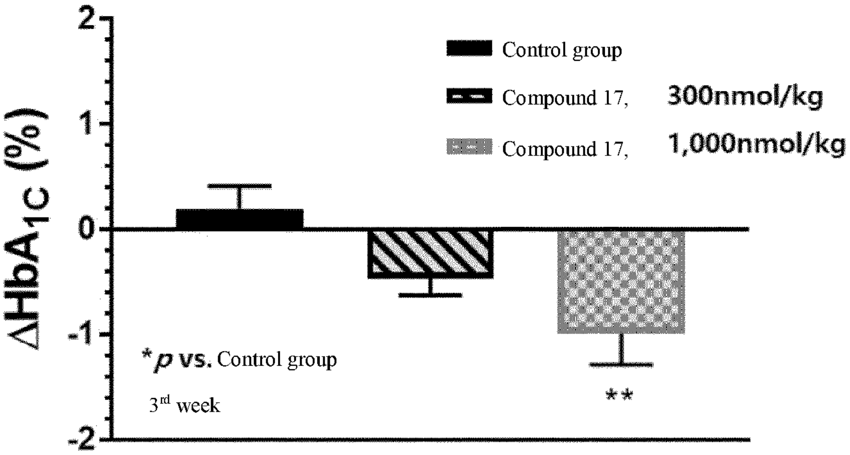


[FIG. 14]

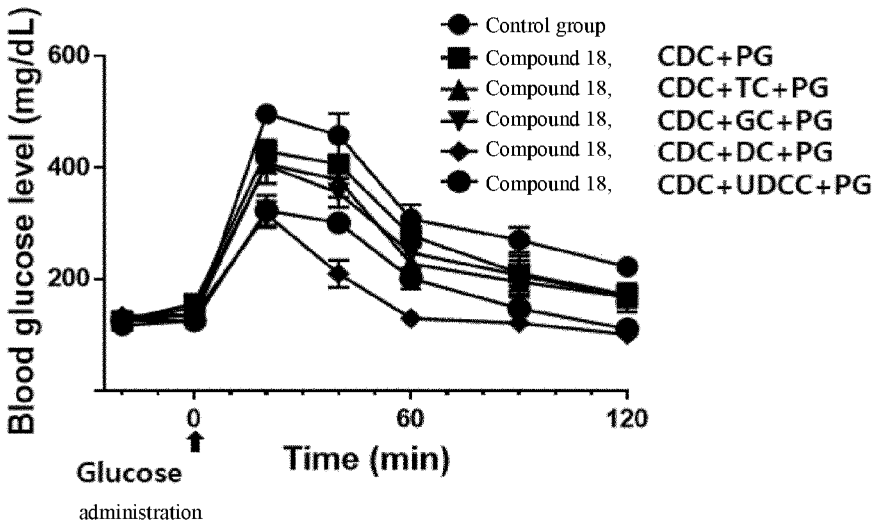


[FIG. 15]

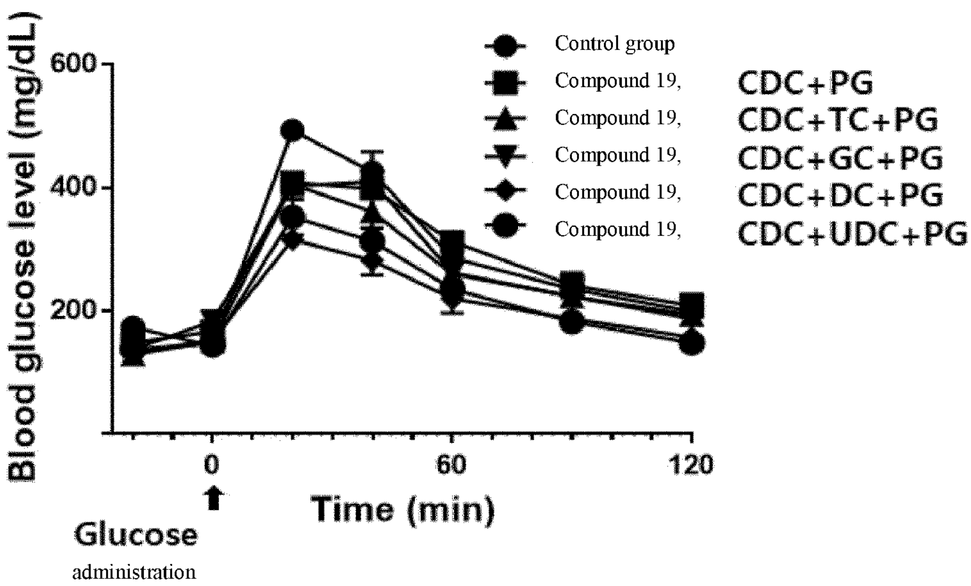
Blood glucose level (mg/dL)
600
400
200
0
60
120
Time (min)
Glucose
administration
Control group
Compound 19, CDC+PG
Compound 19, CDC+TC+PG
Compound 19, CDC+GC+PG
Compound 19, CDC+DC+PG
Compound 19, CDC+UDC+PG

[FIG. 16]

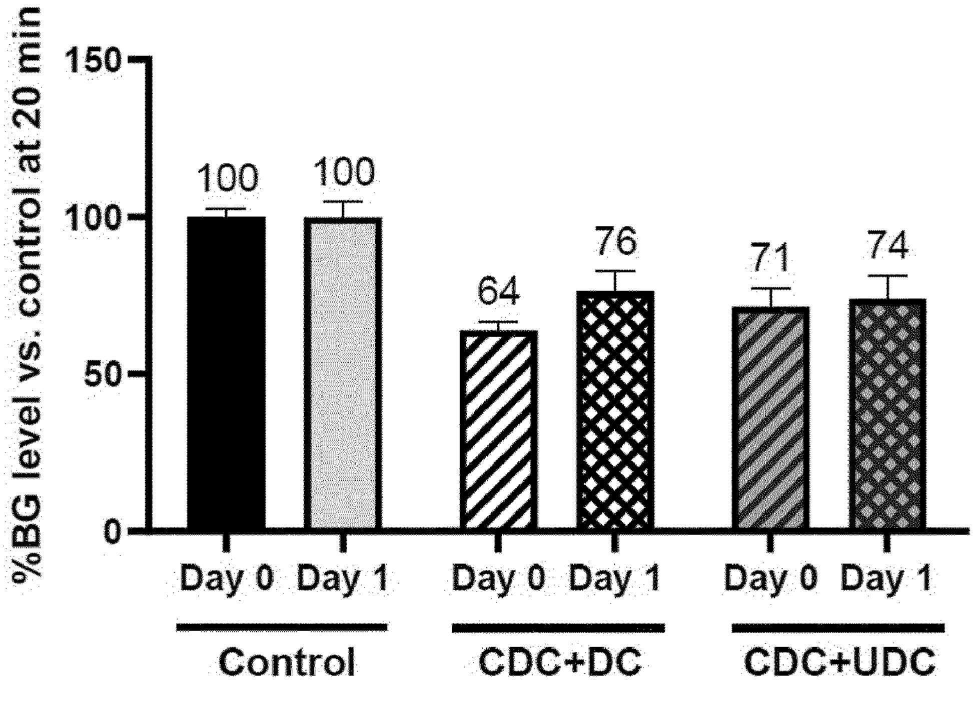
%BG level vs. control at 20 min
150
100
50
0
100
100
64
76
71
74
Day 0
Day 1
Day 0
Day 1
Day 0
Day 1
Control
CDC+DC
CDC+UDC

[FIG. 17]

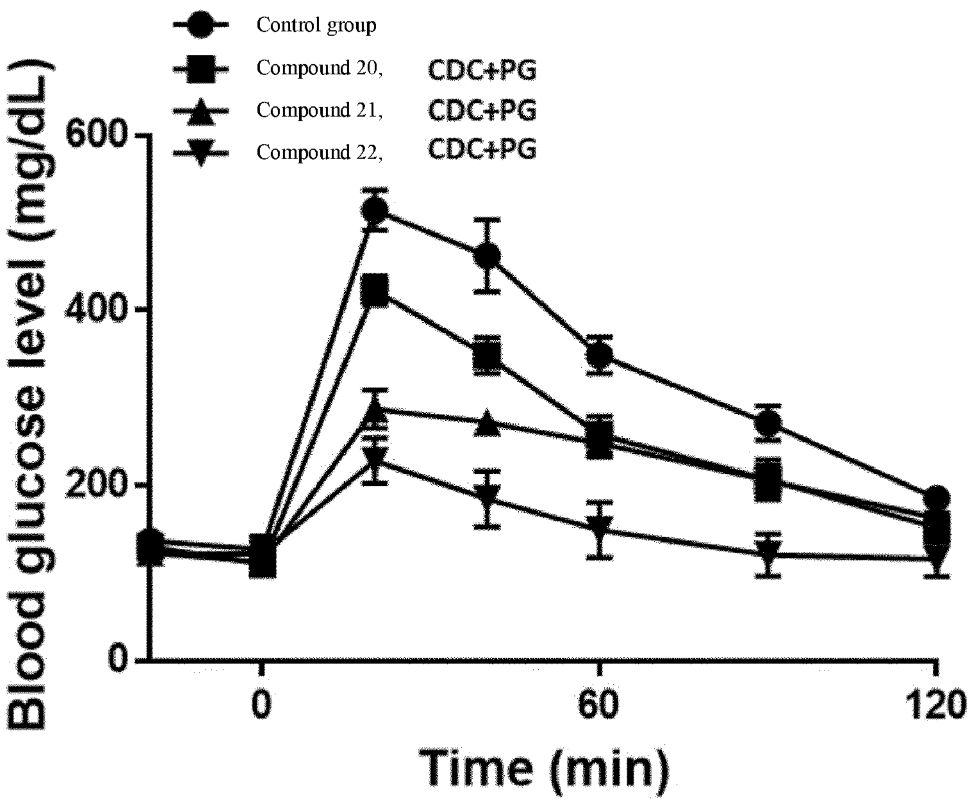
Control group
Compound 20, CDC+PG
Compound 21, CDC+PG
Compound 22, CDC+PG
Blood glucose level (mg/dL)
600
400
200
0
0
60
120
Time (min)

## INTERNATIONAL SEARCH REPORT

International application No.
**PCT/KR2022/013497**

**A. CLASSIFICATION OF SUBJECT MATTER**

**A61K 9/00**(2006.01)i; **A61K 47/28**(2006.01)i; **A61K 38/00**(2006.01)i

According to International Patent Classification (IPC) or to both national classification and IPC

**B. FIELDS SEARCHED**

Minimum documentation searched (classification system followed by classification symbols)

A61K 9/00(2006.01); A61K 38/21(2006.01); A61K 38/28(2006.01); A61K 47/14(2006.01); A61K 47/28(2006.01); A61K 47/48(2006.01); A61K 9/14(2006.01); B82Y 5/00(2011.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 거대분자(macromolecules), 흡수증강제(absorption enhancers), 담즙산 유도체(bile acid derivatives), 프로필 갈레이트(propyl gallate), 경구투여(oral administration)

**C. DOCUMENTS CONSIDERED TO BE RELEVANT**

| Category* | Citation of document, with indication, where appropriate, of the relevant passages | Relevant to claim No. |
|---|---|---|
| X | KR 10-2004-0066177 A (ALZA CORPORATION) 23 July 2004 (2004-07-23)<br>See paragraphs [0009], [0011], [0013], [0020], [0073], [0094] and [0100]; claims 1, 2, 4 and 12; and figures 24 and 39b. | 1-21 |
| X | KR 10-2006-0021293 A (AXCESS LIMITED) 07 March 2006 (2006-03-07)<br>See paragraphs [0006] and [0031]-[0032]; example 1; and claims 1 and 8. | 1-21 |
| X | US 5853748 A (NEW, Roger Randal Charles) 29 December 1998 (1998-12-29)<br>See column 1; and claims 1, 5 and 16-18. | 1-8,20-21 |
| A | KR 10-2002-0083905 A (MEDIPLEX CORP.) 04 November 2002 (2002-11-04)<br>See entire document. | 1-21 |
| A | KR 10-2015-0088861 A (MEDIPLEX CORP. et al.) 03 August 2015 (2015-08-03)<br>See entire document. | 1-21 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 December 2022** | **27 December 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2022/013497**

| Patent document cited in search report | Publication date (day/month/year) | Patent family member(s) | Publication date (day/month/year) |
|---|---|---|---|
| KR 10-2004-0066177 A | 23 July 2004 | EP 1465592 A2 | 13 October 2004 |
| | | JP 2005-519884 A | 07 July 2005 |
| | | US 2003-0198619 A1 | 23 October 2003 |
| | | WO 03-053401 A2 | 03 July 2003 |
| | | WO 2003-053401 A3 | 15 January 2004 |
| KR 10-2006-0021293 A | 07 March 2006 | EP 1613355 A1 | 11 January 2006 |
| | | EP 1613355 B1 | 19 September 2012 |
| | | JP 2006-523663 A | 19 October 2006 |
| | | JP 5047611 B2 | 10 October 2012 |
| | | KR 10-1135784 B1 | 16 April 2012 |
| | | US 2006-0122097 A1 | 08 June 2006 |
| | | US 8314058 B2 | 20 November 2012 |
| | | WO 2004-091667 A1 | 28 October 2004 |
| US 5853748 A | 29 December 1998 | CN 1163573 A | 29 October 1997 |
| | | EP 0769960 A1 | 02 May 1997 |
| | | JP 10-504835 A | 12 May 1998 |
| | | KR 10-1997-0705406 A | 09 October 1997 |
| | | WO 96-06635 A1 | 07 March 1996 |
| KR 10-2002-0083905 A | 04 November 2002 | EP 1383518 A1 | 28 January 2004 |
| | | EP 1383518 A4 | 09 November 2005 |
| | | EP 1385530 A1 | 04 February 2004 |
| | | EP 1385530 A4 | 09 November 2005 |
| | | JP 2002-516355 A | 04 June 2002 |
| | | JP 2004-528366 A | 16 September 2004 |
| | | JP 2004-532851 A | 28 October 2004 |
| | | JP 3541007 B2 | 07 July 2004 |
| | | JP 4084199 B2 | 30 April 2008 |
| | | KR 10-0314496 B1 | 22 November 2001 |
| | | KR 10-0487083 B1 | 03 May 2005 |
| | | KR 10-1999-0087944 A | 27 December 1999 |
| | | KR 10-2002-0085782 A | 16 November 2002 |
| | | US 2002-0010153 A1 | 24 January 2002 |
| | | US 2002-0013292 A1 | 31 January 2002 |
| | | US 2004-0152663 A1 | 05 August 2004 |
| | | US 2004-0220143 A1 | 04 November 2004 |
| | | US 6245753 B1 | 12 June 2001 |
| | | US 6589943 B2 | 08 July 2003 |
| | | US 6656922 B2 | 02 December 2003 |
| | | WO 02-087597 A1 | 07 November 2002 |
| | | WO 02-089820 A1 | 14 November 2002 |
| | | WO 99-61481 A1 | 02 December 1999 |
| KR 10-2015-0088861 A | 03 August 2015 | EP 2925786 A1 | 07 October 2015 |
| | | EP 2925786 A4 | 27 April 2016 |
| | | EP 2925786 B1 | 03 October 2018 |
| | | KR 10-1702251 B1 | 02 February 2017 |
| | | US 10702546 B2 | 07 July 2020 |
| | | US 2015-0290335 A1 | 15 October 2015 |
| | | WO 2014-084421 A1 | 05 June 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020190064370
- KR 1020200163362
- KR 1020200163363
- KR 1020200065484